(19) Europäisches Patentamt
European Patent Office
Office européen des brevets

(11) **EP 4 472 385 A1**

(12) **EUROPEAN PATENT APPLICATION**

(43) Date of publication:
**04.12.2024 Bulletin 2024/49**

(21) Application number: **23176122.2**

(22) Date of filing: **30.05.2023**

(51) International Patent Classification (IPC):
***H10K 85/60*** *(2023.01)* ***C07D 251/24*** *(2006.01)*
*H10K 50/165* *(2023.01)* *H10K 50/16* *(2023.01)*
*H10K 50/18* *(2023.01)*

(52) Cooperative Patent Classification (CPC):
**H10K 85/654;** H10K 50/165; H10K 50/166;
H10K 50/18

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Novaled GmbH**
**01099 Dresden (DE)**

(72) Inventors:
- **GALÁN GARCÍA, Elena**
  **01099 Dresden (DE)**
- **KARPOV, Yevhen**
  **01099 Dresden (DE)**
- **PAVICIC, Domagoj**
  **01099 Dresden (DE)**
- **HUANG, Qiang**
  **01099 Dresden (DE)**

(74) Representative: **Bittner, Thomas L.**
**Boehmert & Boehmert**
**Anwaltspartnerschaft mbB**
**Pettenkoferstrasse 22**
**80336 München (DE)**

(54) **ORGANIC LIGHT EMITTING DEVICE AND DISPLAY DEVICE COMPRISING THE SAME**

(57) The present invention relates to an organic light emitting device and a display device comprising the same.

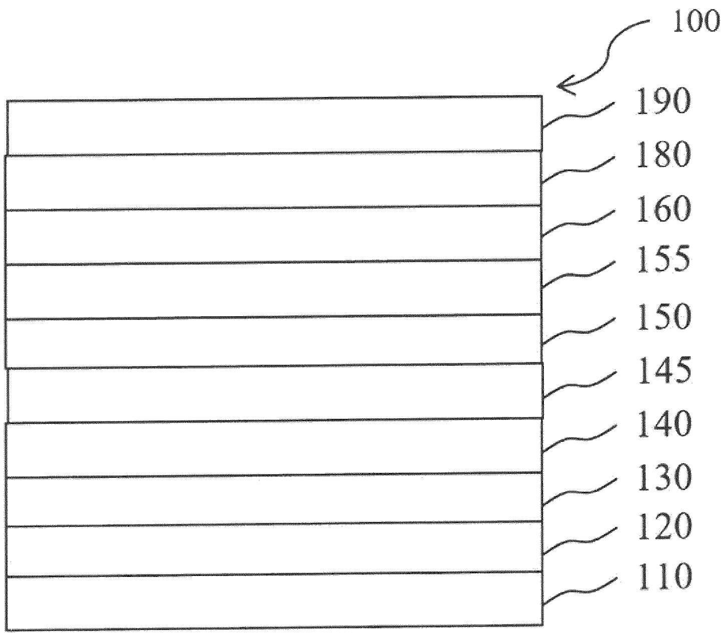


Fig. 1

EP 4 472 385 A1

## Description

The present invention relates to an organic light emitting device and to a display device comprising the organic light emitting device.

## BACKGROUND OF THE INVENTION

Organic semiconducting devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic semiconducting device, such as an organic light emitting diode, may be applied to a large-size flat panel display.

It is, therefore, the object of the present invention to provide organic light emitting diodes and compounds for preparing the same overcoming drawbacks of the prior art, in particular providing compounds for use in organic light emitting diodes comprising the same helpful to improve the performance thereof, especially with respect to efficiency

## DISCLOSURE

This object is achieved by an organic light emitting device comprising an anode, a cathode, an electron transport layer, an emission layer, and an auxiliary electron transport layer,
wherein

- the electron transport layer and the auxiliary electron transport layer are arranged between emission layer and the cathode;

- the auxiliary electron transport layer is arranged between the electron transport layer and the emission layer;

- the electron transport layer comprises a compound of formula (I)

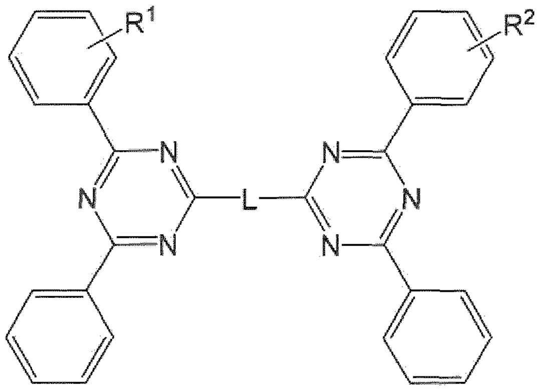

(I)

- $R_1$ and $R_2$ are independently selected from the group consisting of H, substituted or unsubstituted $C_6$ to $C_{18}$ aryl; substituted or unsubstituted $C_3$ to $C_{17}$ heteroaryl, CN, and CN-substituted $C_6$ to $C_{18}$ aryl;

  - wherein if one or more of $R_1$ and $R^2$ is substituted $C_6$ to $C_{18}$ aryl or $C_3$ to $C_{17}$ heteroaryl, the one or more substituent(s) are independently selected from the group consisting of D and $C_1$ to $C_4$ alkyl;

- L is selected from formulas (a) to (q)

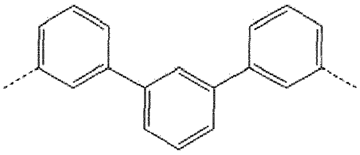
(a)
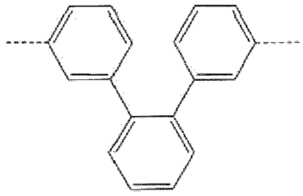
(b)
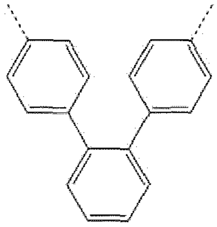
(c)
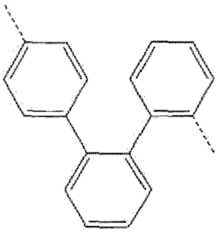
(d)
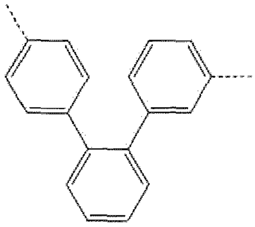
(e)
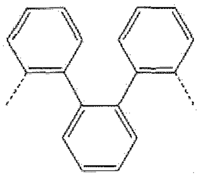
(f)
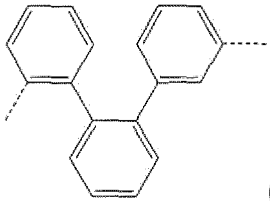
(g)
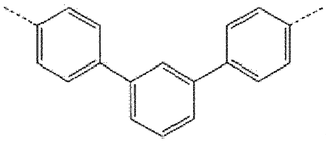
(h)
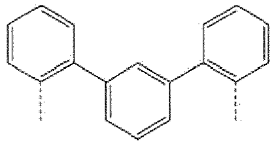
(i)
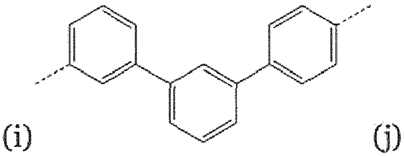
(j)
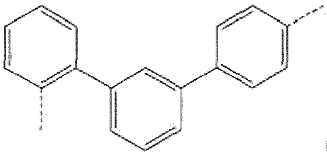
(k)
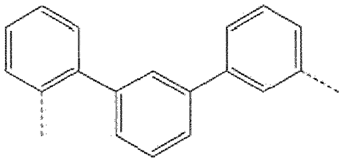
(l)
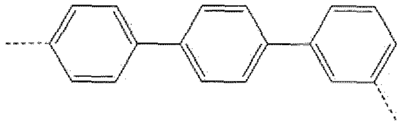
(m)
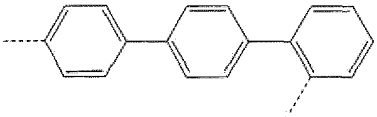
(n)
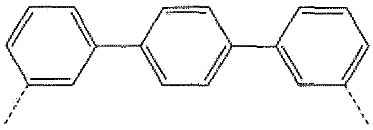
(o)
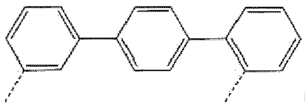
(p)
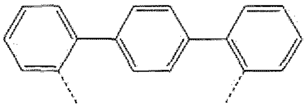
(q);

and

- the electron transport layer further comprises an alkali metal complex.

The object is further achieved by a display device comprising the organic light emitting device according to the present invention.

Surprisingly, it was found that the organic light emitting device according to the invention has improved properties in comparison with respective devices of the prior art, especially with improved (higher) current efficiency and improved (longer) lifetime.

Compound of formula (I)

The organic light emitting device comprises in an electron transport layer thereof a compound of Formula (I).

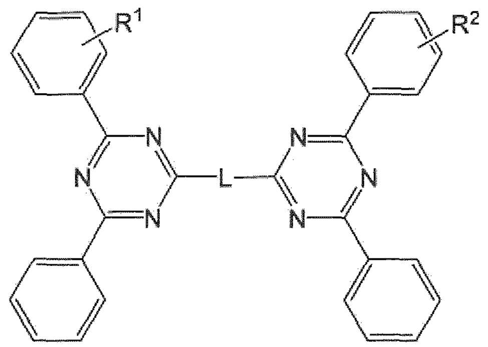

(I).

Especially, if not mentioned else explicitly, all compounds, especially the compound of formula (I), groups, moieties, substituents etc. shown herein, especially by structural formulas, by systematic names etc. encompass the respective partially and fully deuterated derivatives thereof, wherein it may be provided that substitution beyond deuteration is excluded.

In accordance with the present disclosure, as an illustrative example using an arbitrary group A in a formula showing the following binding situation,

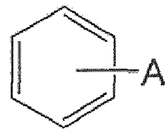

the group A may be bound to any suitable binding position. That is, in formula (I) $R_1$ may bonded to the positions marked with the *-symbol in the following formula, respectively

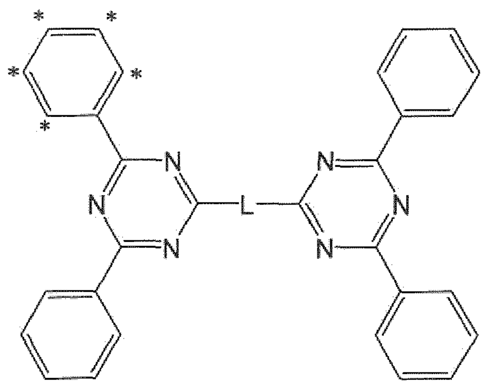

;

and in formula (I) $R^2$ may bonded to the positions marked with the *-symbol in the following formula, respectively

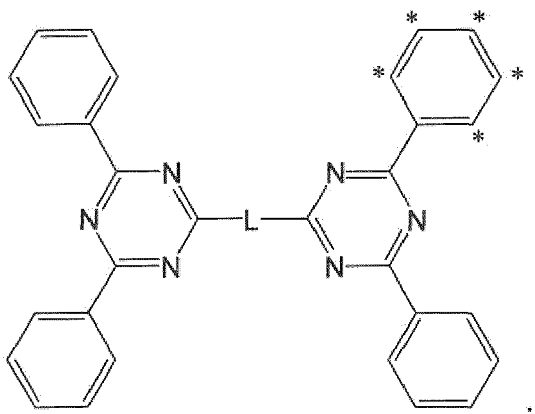

.

It is provided that the compound of formula (I) comprises exactly one group $R^1$ and exactly one group $R^2$. If one or

both of $R^1$ and $R^2$ are H, the remaining H bonded to the respective phenyl ring are not considered as $R^1$, respectively $R^2$. In other words, the fact that in case that $R^1$ and/or $R^2$ is/are H more than one H is attached to the respective phenyl ring is not in conflict with the provision that the compound of formula (I) comprises exactly one group $R^1$ and exactly one group $R^2$ since only one of the H is considered as $R^1$/$R^2$.

$R^1$ and $R^2$ are independently selected from the group consisting of H, substituted or unsubstituted $C_6$ to $C_{18}$ aryl; substituted or unsubstituted $C_3$ to $C_{17}$ heteroaryl, CN, and CN-substituted $C_6$ to $C_{18}$ aryl. $R^1$ and $R^2$ may be independently selected from the group consisting of H, substituted or unsubstituted $C_6$ to $C_{12}$ aryl; substituted or unsubstituted $C_3$ to $C_{11}$ heteroaryl, CN, and CN-substituted $C_6$ to $C_{12}$ aryl. $R^1$ and $R^2$ may be independently selected from the group consisting of H, substituted or unsubstituted $C_6$ to $C_{10}$ aryl; substituted or unsubstituted $C_3$ to $C_9$ heteroaryl, CN, and CN-substituted $C_6$ to $C_{10}$ aryl. $R^1$ and $R^2$ may be independently selected from the group consisting of H, substituted or unsubstituted phenyl, substituted or unsubstituted naphthyl, substituted or unsubstituted pyridyl, substituted or unsubstituted quinolinyl, CN, and CN-substituted phenyl. $R^1$ and $R^2$ may be independently selected from the group consisting of H and substituted or unsubstituted phenyl. $R^1$ and $R^2$ may both be H.

$R^1$ may be H and $R^2$ may be selected from the group consisting of H, substituted or unsubstituted $C_6$ to $C_{18}$ aryl; substituted or unsubstituted $C_3$ to $C_{17}$ heteroaryl, CN, and CN-substituted $C_6$ to $C_{18}$ aryl. $R^1$ may be H and $R^2$ may be selected from the group consisting of H, substituted or unsubstituted $C_6$ to $C_{12}$ aryl; substituted or unsubstituted $C_3$ to $C_{11}$ heteroaryl, CN, and CN-substituted $C_6$ to $C_{12}$ aryl. $R^1$ may be H and $R^2$ may be selected from the group consisting of H, substituted or unsubstituted $C_6$ to $C_{10}$ aryl; substituted or unsubstituted $C_3$ to $C_9$ heteroaryl, CN, and CN-substituted $C_6$ to $C_{10}$ aryl. $R^1$ may be H and $R^2$ may be selected from the group consisting of H, substituted or unsubstituted phenyl, substituted or unsubstituted naphthyl, substituted or unsubstituted pyridyl, substituted or unsubstituted quinolinyl, CN, and CN-substituted phenyl. $R^1$ may be H and $R^2$ may be H or substituted or unsubstituted phenyl. $R^1$ may be H and $R^2$ may be substituted or unsubstituted phenyl. $R^1$ and $R^2$ may both be H.

If one or more of $R^1$ and $R^2$ is substituted $C_6$ to $C_{18}$ aryl or $C_3$ to $C_{17}$ heteroaryl, the one or more substituent(s) are independently selected from the group consisting of D and $C_1$ to $C_4$ alkyl. If one or more of $R^1$ and $R^2$ is substituted $C_6$ to $C_{18}$ aryl or $C_3$ to $C_{17}$ heteroaryl, the one or more substituent(s) may be independently selected from the group consisting of D and $C_1$ to $C_2$ alkyl. If one or more of $R^1$ and $R^2$ is substituted $C_6$ to $C_{18}$ aryl or $C_3$ to $C_{17}$ heteroaryl, the one or more substituent(s) may be independently selected from the group consisting of D and methyl. If one or more of $R^1$ and $R^2$ is substituted $C_6$ to $C_{18}$ aryl or $C_3$ to $C_{17}$ heteroaryl, the one or more substituent(s) may be each D (deuterium).

$R^1$ and $R^2$ may be independently selected from the group consisting of H, $C_6$ to $C_{18}$ aryl; $C_3$ to $C_{17}$ heteroaryl, CN, and CN-substituted $C_6$ to $C_{18}$ aryl. $R^1$ and $R^2$ may be independently selected from the group consisting of H, $C_6$ to $C_{12}$ aryl; $C_3$ to $C_{11}$ heteroaryl, CN, and CN-substituted $C_6$ to $C_{12}$ aryl. $R_1$ and $R^2$ may be independently selected from the group consisting of H, $C_6$ to $C_{10}$ aryl; $C_3$ to $C_9$ heteroaryl, CN, and CN-substituted $C_6$ to $C_{10}$ aryl. $R^1$ and $R^2$ may be independently selected from the group consisting of H, phenyl, naphthyl, pyridyl, quinolinyl, CN, and CN-substituted phenyl. $R^1$ and $R^2$ may be independently selected from the group consisting of H and phenyl. $R^1$ and $R^2$ may both be H.

$R^1$ may be H and $R^2$ may be selected from the group consisting of H, $C_6$ to $C_{18}$ aryl; $C_3$ to $C_{17}$ heteroaryl, CN, and CN-substituted $C_6$ to $C_{18}$ aryl. $R^1$ may be H and $R^2$ may be selected from the group consisting of H, $C_6$ to $C_{12}$ aryl; $C_3$ to $C_{11}$ heteroaryl, CN, and CN-substituted $C_6$ to $C_{12}$ aryl. $R^1$ may be H and $R^2$ may be selected from the group consisting of H, $C_6$ to $C_{10}$ aryl; $C_3$ to $C_9$ heteroaryl, CN, and CN-substituted $C_6$ to $C_{10}$ aryl. $R^1$ may be H and $R^2$ may be selected from the group consisting of H, phenyl, naphthyl, pyridyl, quinolinyl, CN, and CN-substituted phenyl. $R^1$ may be H and $R^2$ may be H or phenyl. $R^1$ may be H and $R^2$ may be phenyl. $R^1$ and $R^2$ may both be H.

L is selected from formulas (a) to (q)

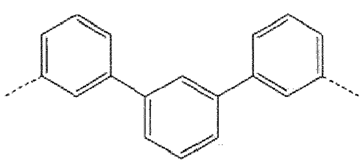
(a)

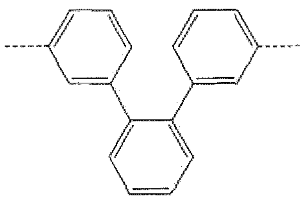
(b)

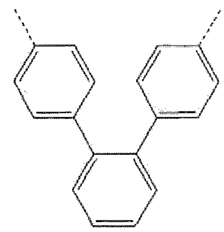
(c)

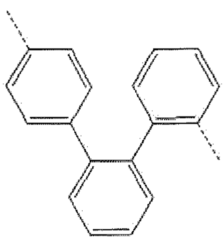
(d)

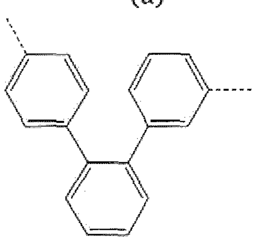
(e)

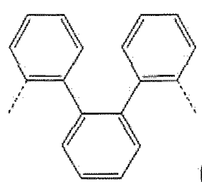
(f)

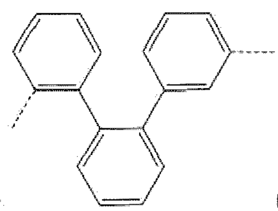
(g)

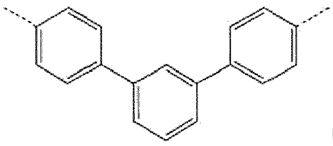 (h) 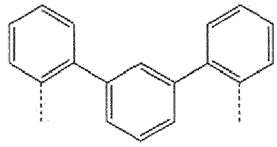 (i) 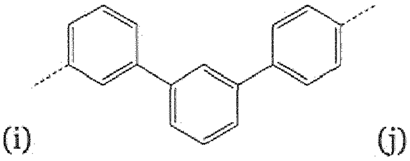 (j)

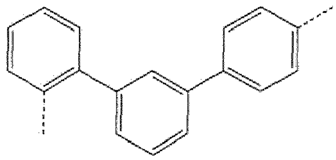 (k) 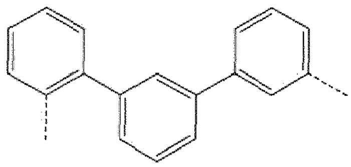 (l) 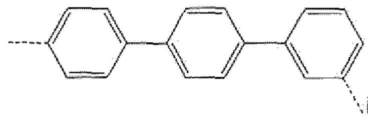 (m)

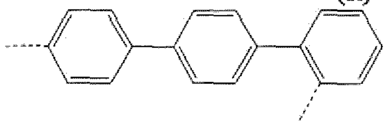 (n) 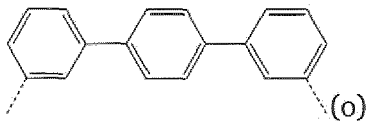 (o) 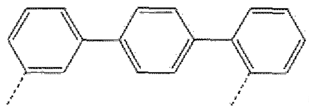 (p)

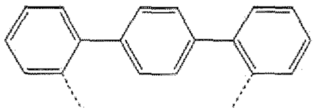 (q).

In the formulas (a) to (q), the dotted lines "------" represent the binding positions to

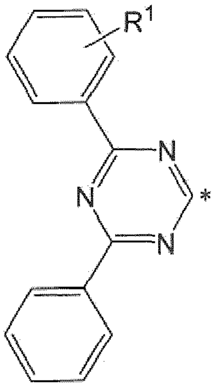 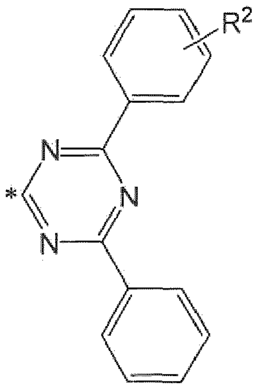

;

at the positions labeled with the *, respectively.

The compound of formula (I) may have a calculated HOMO value of from -5,85 eV to -6,20 eV, from -5,90 eV to -6,10 eV, or from -6,02 eV to -6,07 eV.

The compound of formula (I) may have a calculated LUMO value of from -1,70 eV to -1,95 eV, from -1,80 eV to -1,95 eV, or from -1,82 eV to -1,92 eV.

The compound of formula (I) may have a calculated $E_{gap}$ (LUMO - HOMO) from 4,00 eV to 4, 40 eV, from 4,10 eV to 4,30 eV, from 4,11 eV to 4,26 eV.

The compound of formula (I) may have a melting point from 260°C to 350 °C, or from 278 °C to 348°C.

The compound of formula (I) may have a glass transition temperature Tg from 110°C to 138°C.

The compound of formula (I) may have a rate onset from 220°C to 310°C or from 234°C to 284°C.

For example, if L has the formula (e), the compound of formula (I) has the formula

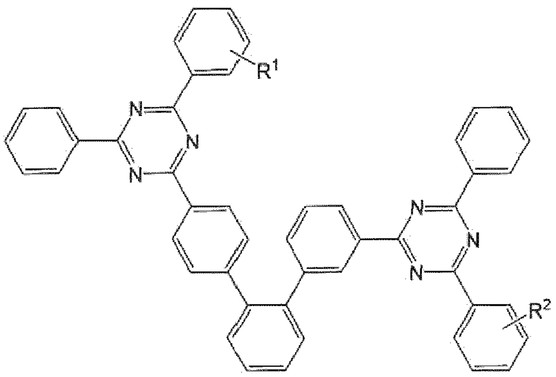

or

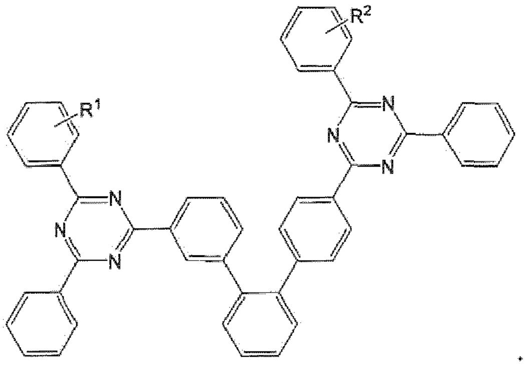

L may be selected from (a) to (e)

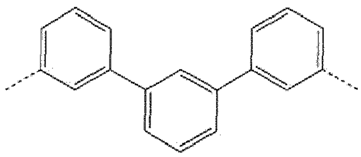 (a)

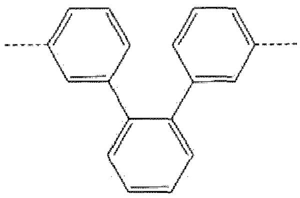 (b)

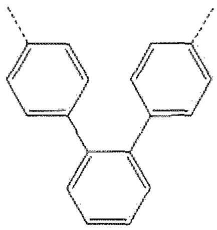 (c)

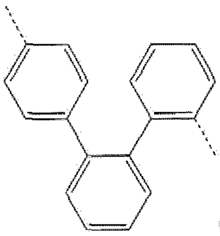 (d)

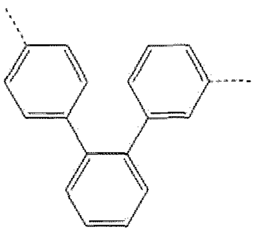 (e).

In one embodiment, there is provided an organic light emitting device comprising an anode, a cathode, an electron transport layer, an emission layer, and an auxiliary electron transport layer,
wherein

- the electron transport layer and the auxiliary electron transport layer are arranged between emission layer and the cathode;
- the auxiliary electron transport layer is arranged between the electron transport layer and the emission layer;
- the electron transport layer comprises a compound of formula (I)

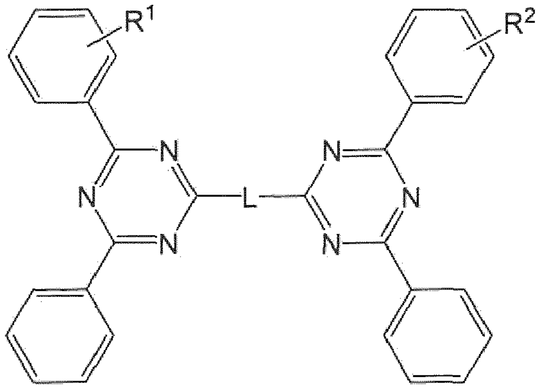

(I)

- $R^1$ and $R^2$ are independently selected from the group consisting of H, substituted or unsubstituted phenyl, substituted or unsubstituted naphthyl, substituted or unsubstituted pyridyl, substituted or unsubstituted quinolinyl, CN, and CN-substituted phenyl;

  - wherein if one or more of $R^1$ and $R^2$ is substituted $C_6$ to $C_{18}$ aryl or $C_3$ to $C_{17}$ heteroaryl, the one or more substituent(s) are independently selected from the group consisting of D and $C_1$ to $C_4$ alkyl;

- L is selected from formulas (a) to (q)

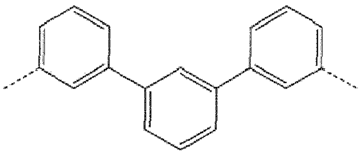 (a) 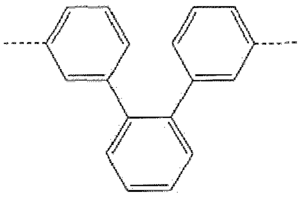 (b) 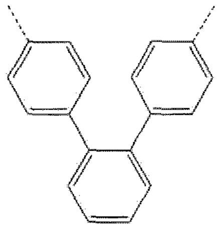 (c)

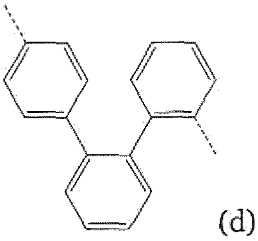 (d) 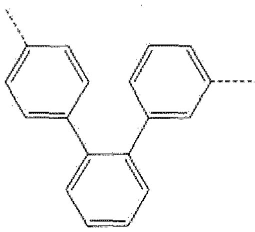 (e) 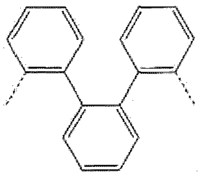 (f) 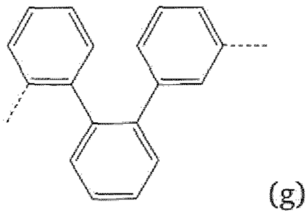 (g)

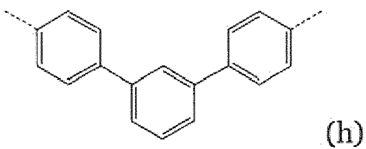 (h) 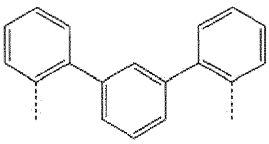 (i) 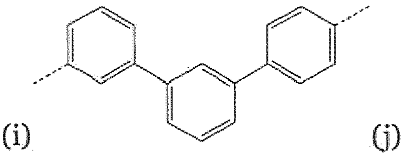 (j)

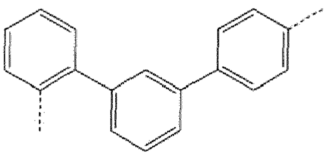 (k) 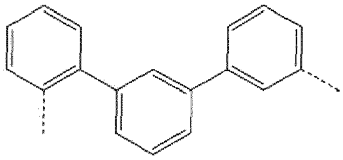 (l) 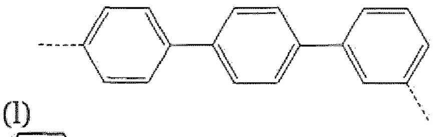 (m)

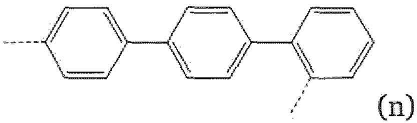 (n) 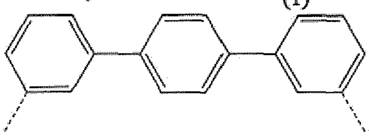 (o) 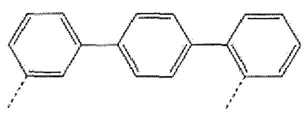 (p)

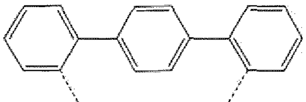 (q);

and

- the electron transport layer further comprises a lithium metal complex.

In one embodiment, there is provided an organic light emitting device comprising an anode, a cathode, an electron transport layer, an emission layer, and an auxiliary electron transport layer,
wherein

- the electron transport layer and the auxiliary electron transport layer are arranged between emission layer and the cathode;

- the auxiliary electron transport layer is arranged between the electron transport layer and the emission layer;

- the electron transport layer comprises a compound of formula (I)

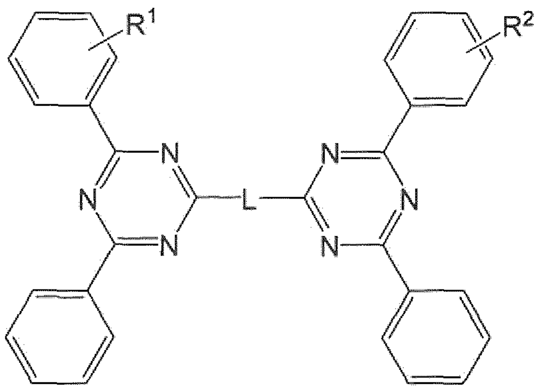

(I)

- $R^1$ and $R^2$ are independently selected from the group consisting of H, phenyl, naphthyl, pyridyl, quinolinyl, CN, and CN-substituted phenyl;

- L is selected from formulas (a) to (e)

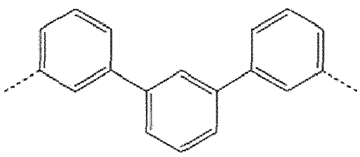

(a)

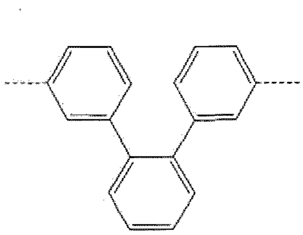

(b)

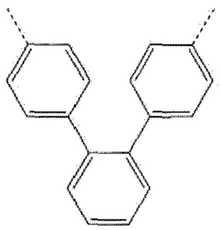

(c)

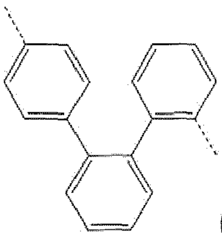

(d)

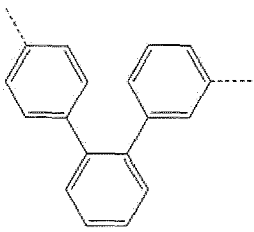

(e);

and

- the electron transport layer further comprises 8-hydroxyquinolinolato-lithium.

The compound of Formula I may be selected from I-1 to I-25

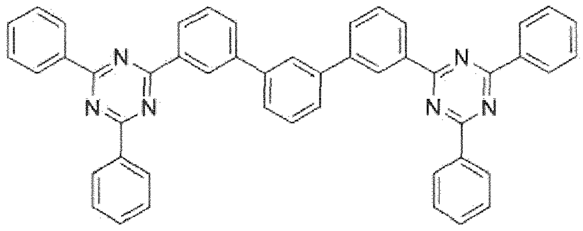

(I-1);

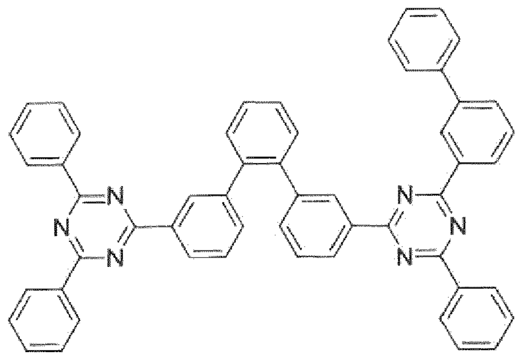

(I-2);

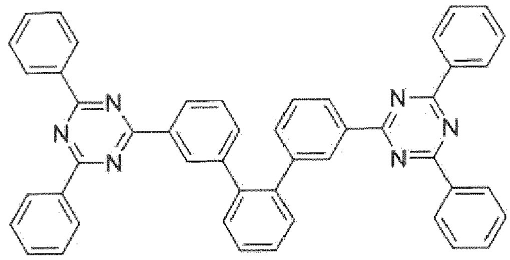

(I-3);

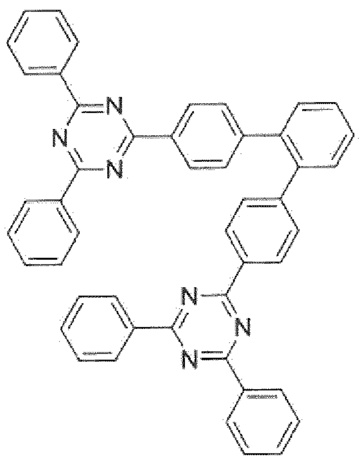

(I-4);

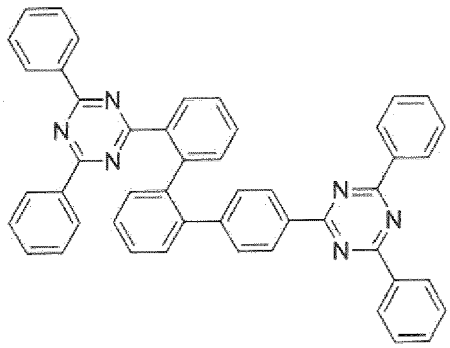

(I-5);

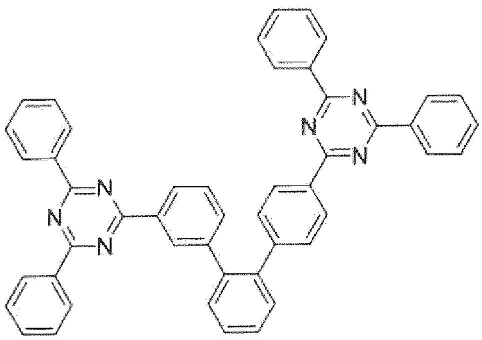

(I-6);

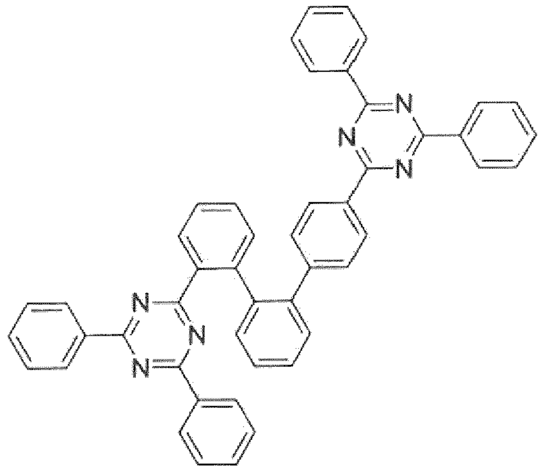

(I-7)

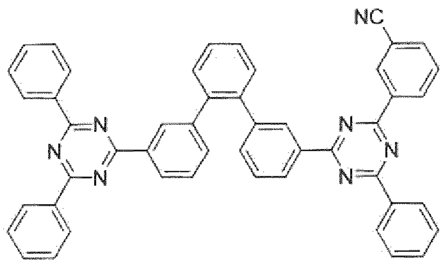

(I-8)

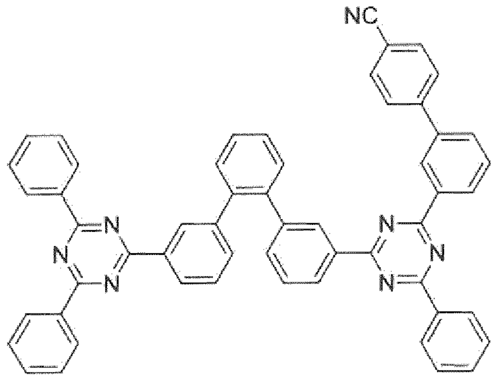

(I-9)

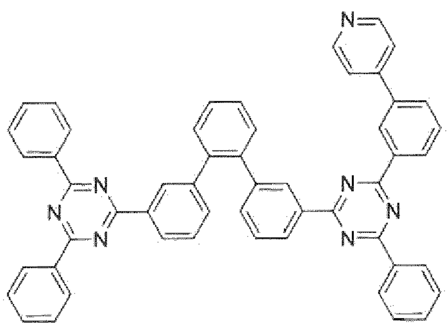

(I-10)

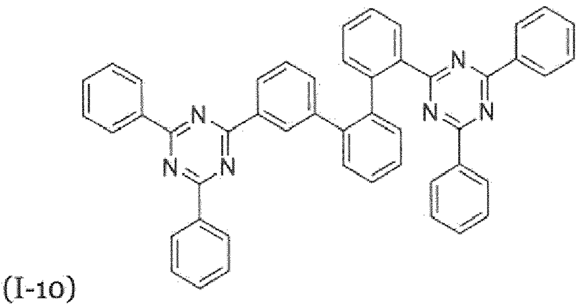

(I-11)

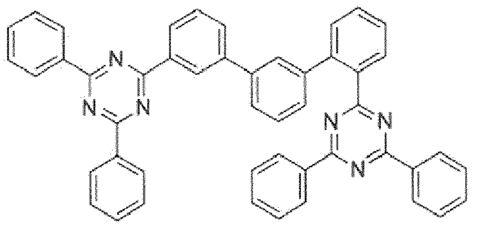

(I-12)

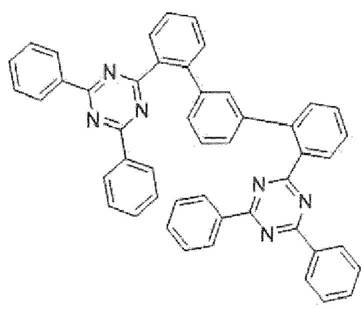

(I-13)

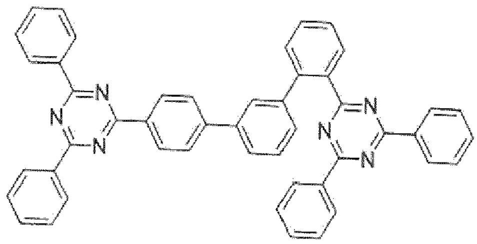

(I-14)

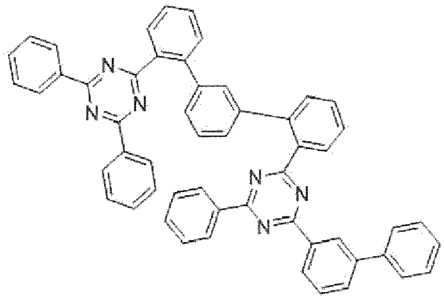

(I-15)

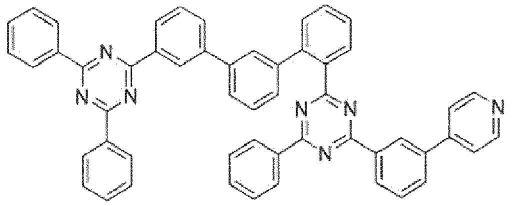

(I-16)

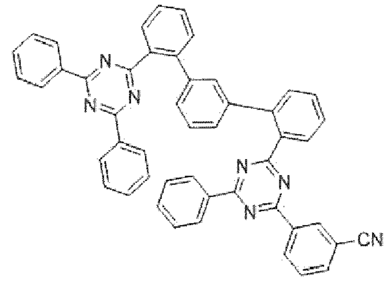

(I-17)

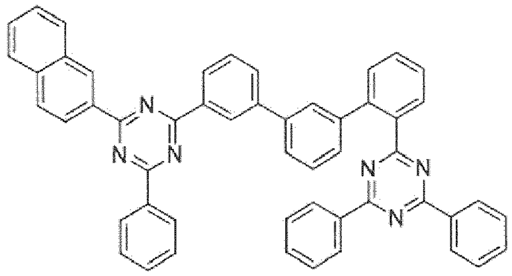

(I-18)

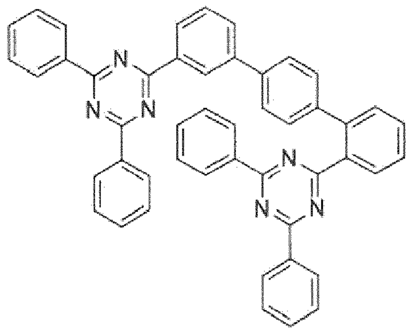

(I-19)

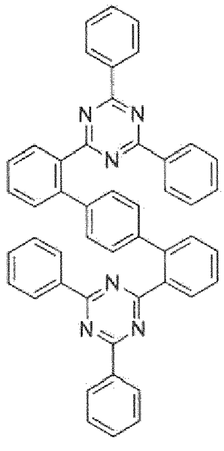

(I-20)

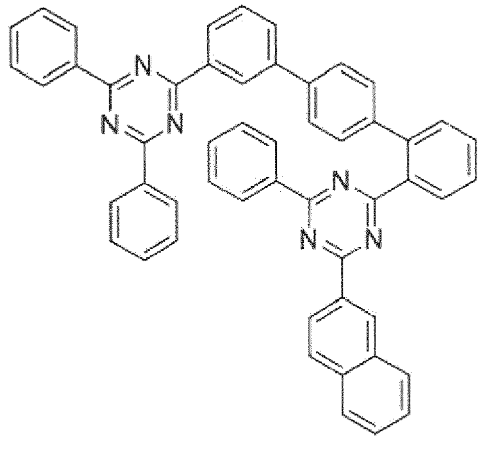

(I-21)

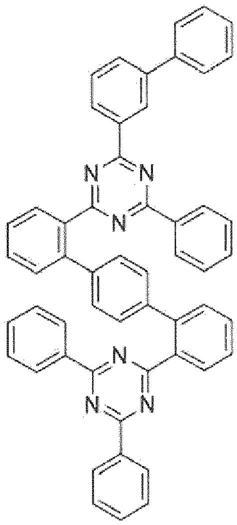
(I-22)

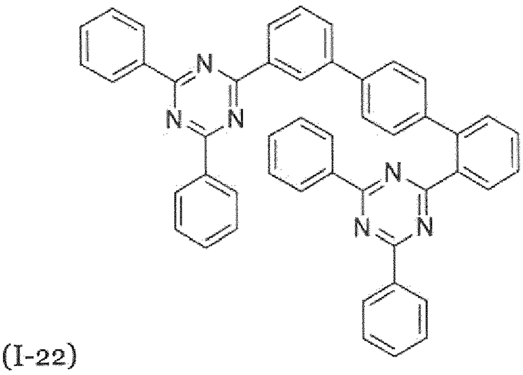
(I-23)

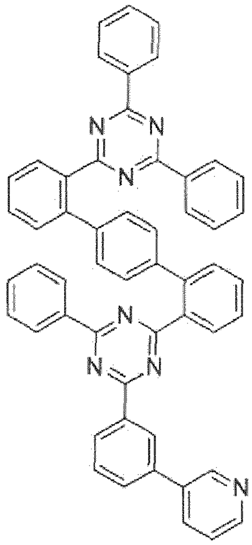
(I-24)

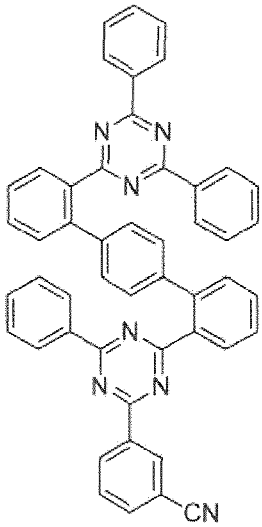
(I-25).

The compound of Formula I may be selected from I-1 to 1-6

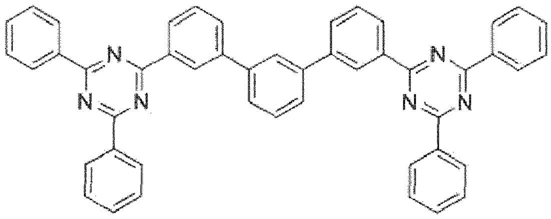
(I-1);

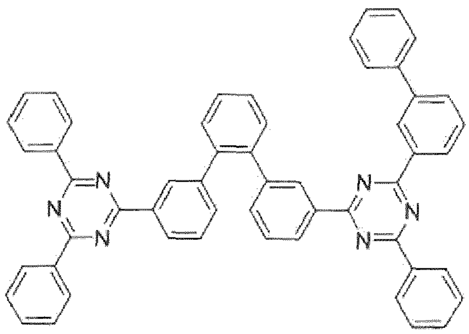
(I-2);

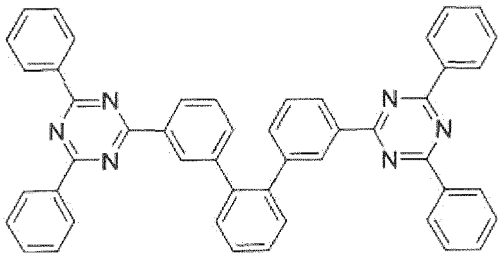
(I-3);

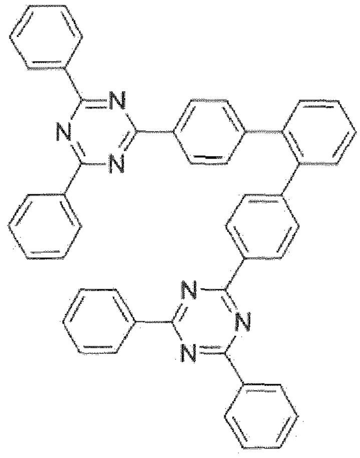
(I-4);

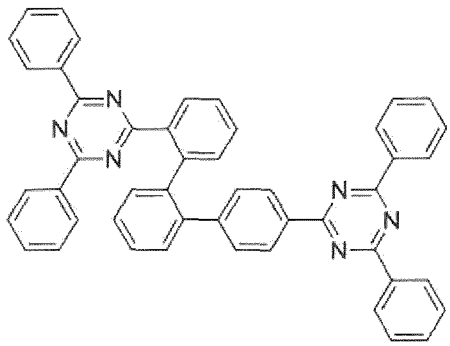
(I-5);

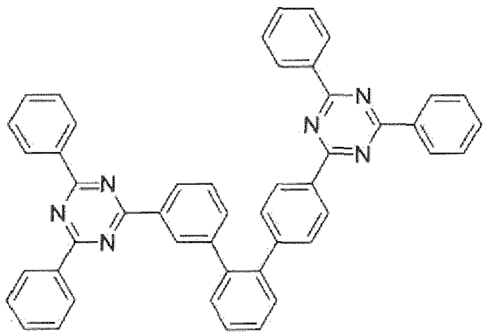
(I-6).

According to one embodiment of the organic light emitting device according to the invention, there is provided an organic light emitting device comprising an anode, a cathode, an electron transport layer, an emission layer, and an auxiliary electron transport layer,
wherein

- the electron transport layer and the auxiliary electron transport layer are arranged between emission layer and the cathode;
- the auxiliary electron transport layer is arranged between the electron transport layer and the emission layer;
- the electron transport layer comprises a compound of formula (II)

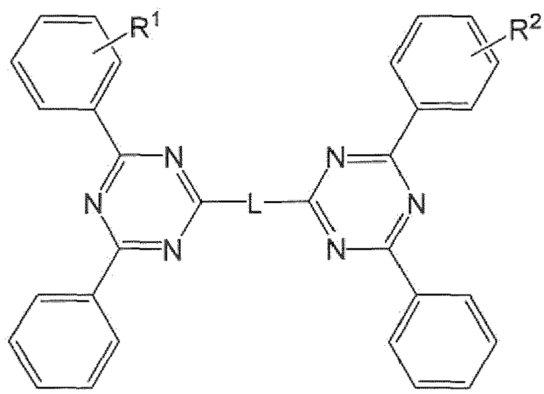
(II)

- $R^1$ and $R^2$ are independently selected from the group consisting of H, substituted or unsubstituted $C_6$ to $C_{18}$ aryl; substituted or unsubstituted $C_3$ to $C_{17}$ heteroaryl, CN, and CN-substituted $C_6$ to $C_{18}$ aryl;

- wherein if one or more of $R^1$ and $R^2$ is substituted $C_6$ to $C_{18}$ aryl or $C_3$ to $C_{17}$ heteroaryl, the one or more substituent(s) are independently selected from the group consisting of D and $C_1$ to $C_4$ alkyl;

- L is selected from formulas (a), and (h) to (l)

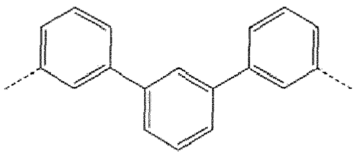
(a)
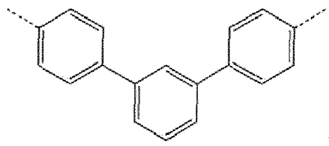
(h)
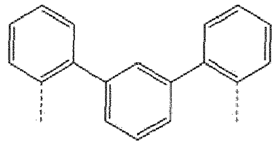
(i
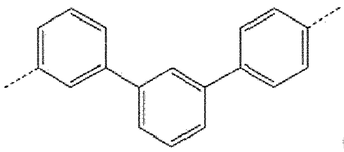
(j)
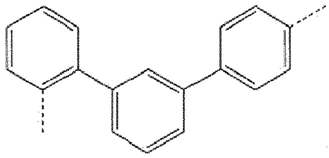
(k)
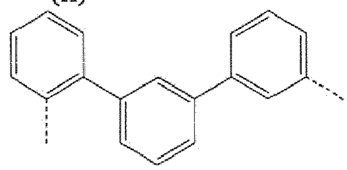
(l)

and

- the electron transport layer further comprises an alkali metal complex.

This embodiment encompasses layer structures, device architectures etc. described herein, especially described with respect to devices comprising the compound of formula (I), wherein the compound of formula (I) is replaced by the compound of formula (II).

The organic light emitting device comprises in an electron transport layer thereof a compound of Formula (II).

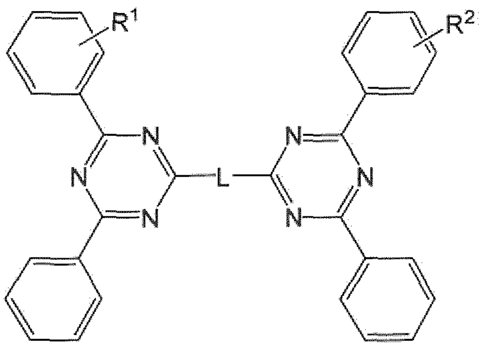
(II).

Especially, if not mentioned else explicitly, all compounds, especially the compound of formula (II), groups, moieties, substituents etc. shown herein, especially by structural formulas, by systematic names etc. encompass the respective partially and fully deuterated derivatives thereof, wherein it may be provided that substitution beyond deuteration is excluded.

In accordance with the present disclosure, as an illustrative example using an arbitrary group A in a formula showing the following binding situation,

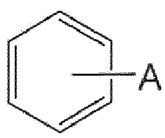

the group A may be bound to any suitable binding position. That is, in formula (II) $R^1$ may bonded to the positions marked with the *-symbol in the following formula, respectively

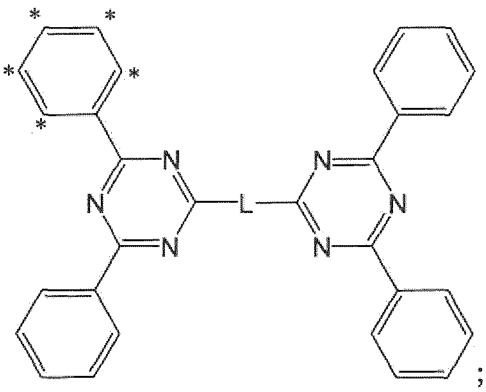

;

and in formula (II) $R^2$ may bonded to the positions marked with the *-symbol in the following formula, respectively

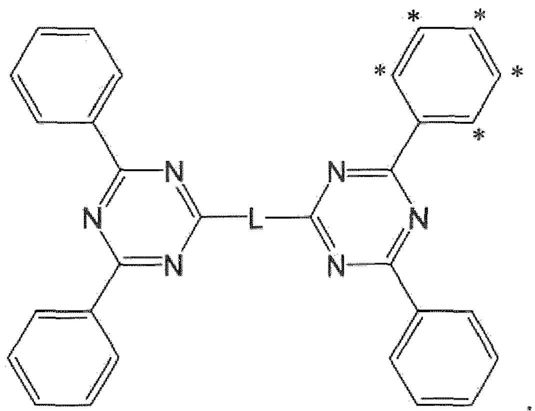

.

It is provided that the compound of formula (II) comprises exactly one group $R^1$ and exactly one group $R^2$. If one or both of $R^1$ and $R^2$ are H, the remaining H bonded to the respective phenyl ring are not considered as $R^1$, respectively $R^2$. In other words, the fact that in case that $R^1$ and/or $R^2$ is/are H more than one H is attached to the respective phenyl ring is not in conflict with the provision that the compound of formula (II) comprises exactly one group $R^1$ and exactly one group $R^2$ since only one of the H is considered as $R^1$/$R^2$.

In formula (II), $R^1$ and $R^2$ are independently selected from the group consisting of H, substituted or unsubstituted $C_6$ to C,s aryl; substituted or unsubstituted $C_3$ to $C_{17}$ heteroaryl, CN, and CN-substituted $C_6$ to $C_{18}$ aryl. $R^1$ and $R^2$ may be independently selected from the group consisting of H, substituted or unsubstituted $C_6$ to $C_{12}$ aryl; substituted or unsubstituted $C_3$ to $C_{11}$ heteroaryl, CN, and CN-substituted $C_6$ to $C_{12}$ aryl. $R^1$ and $R^2$ may be independently selected from the group consisting of H, substituted or unsubstituted $C_6$ to $C_{10}$ aryl; substituted or unsubstituted $C_3$ to $C_9$ heteroaryl, CN, and CN-substituted $C_6$ to $C_{10}$ aryl. $R^1$ and $R^2$ may be independently selected from the group consisting of H, substituted or unsubstituted phenyl, substituted or unsubstituted naphthyl, substituted or unsubstituted pyridyl, substituted or unsubstituted quinolinyl, CN, and CN-substituted phenyl. $R^1$ and $R^2$ may be independently selected from the group consisting of H and substituted or unsubstituted phenyl. $R^1$ and $R^2$ may both be H.

In formula (II), $R^1$ may be H and $R^2$ may be selected from the group consisting of H, substituted or unsubstituted $C_6$ to Cis aryl; substituted or unsubstituted $C_3$ to $C_{17}$ heteroaryl, CN, and CN-substituted $C_6$ to $C_{18}$ aryl. $R^1$ may be H and $R^2$ may be selected from the group consisting of H, substituted or unsubstituted $C_6$ to $C_{12}$ aryl; substituted or unsubstituted $C_3$ to $C_{11}$ heteroaryl, CN, and CN-substituted $C_6$ to $C_{12}$ aryl. $R^1$ may be H and $R^2$ may be selected from the group consisting of H, substituted or unsubstituted $C_6$ to $C_{10}$ aryl; substituted or unsubstituted $C_3$ to $C_9$ heteroaryl, CN, and CN-substituted $C_6$ to $C_{10}$ aryl. $R^1$ may be H and $R^2$ may be selected from the group consisting of H, substituted or unsubstituted phenyl, substituted or unsubstituted naphthyl, substituted or unsubstituted pyridyl, substituted or unsubstituted quinolinyl, CN, and CN-substituted phenyl. $R^1$ may be H and $R^2$ may be H or substituted or unsubstituted phenyl. $R^1$ may be H and $R^2$ may be substituted or unsubstituted phenyl. $R^1$ and $R^2$ may both be H.

In formula (II), if one or more of $R^1$ and $R^2$ is substituted $C_6$ to $C_{18}$ aryl or $C_3$ to $C_{17}$ heteroaryl, the one or more substituent(s) are independently selected from the group consisting of D and $C_1$ to $C_4$ alkyl. If one or more of $R^1$ and $R^2$ is substituted $C_6$ to $C_{18}$ aryl or $C_3$ to $C_{17}$ heteroaryl, the one or more substituent(s) may be independently selected from the group consisting of D and $C_1$ to $C_2$ alkyl. If one or more of $R^1$ and $R^2$ is substituted $C_6$ to $C_{18}$ aryl or $C_3$ to $C_{17}$ heteroaryl, the one or more substituent(s) may be independently selected from the group consisting of D and methyl. If one or more of $R^1$

and $R^2$ is substituted $C_6$ to $C_{18}$ aryl or $C_3$ to $C_{17}$ heteroaryl, the one or more substituent(s) may be each D (deuterium).

In formula (II), $R^1$ and $R^2$ may be independently selected from the group consisting of H, $C_6$ to $C_{18}$ aryl; $C_3$ to $C_{17}$ heteroaryl, CN, and CN-substituted $C_6$ to $C_{18}$ aryl. $R^1$ and $R^2$ may be independently selected from the group consisting of H, $C_6$ to $C_{12}$ aryl; $C_3$ to $C_{11}$ heteroaryl, CN, and CN-substituted $C_6$ to $C_{12}$ aryl. $R^1$ and $R^2$ may be independently selected from the group consisting of H, $C_6$ to $C_{10}$ aryl; $C_3$ to $C_9$ heteroaryl, CN, and CN-substituted $C_6$ to $C_{10}$ aryl. $R^1$ and $R^2$ may be independently selected from the group consisting of H, phenyl, naphthyl, pyridyl, quinolinyl, CN, and CN-substituted phenyl. $R^1$ and $R^2$ may be independently selected from the group consisting of H and phenyl. $R^1$ and $R^2$ may both be H.

In formula (II), $R^1$ may be H and $R^2$ may be selected from the group consisting of H, $C_6$ to $C_{18}$ aryl; $C_3$ to $C_{17}$ heteroaryl, CN, and CN-substituted $C_6$ to $C_{18}$ aryl. $R^1$ may be H and $R^2$ may be selected from the group consisting of H, $C_6$ to $C_{12}$ aryl; $C_3$ to $C_{11}$ heteroaryl, CN, and CN-substituted $C_6$ to $C_{12}$ aryl. $R^1$ may be H and $R^2$ may be selected from the group consisting of H, $C_6$ to $C_{10}$ aryl; $C_3$ to $C_9$ heteroaryl, CN, and CN-substituted $C_6$ to $C_{10}$ aryl. $R^1$ may be H and $R^2$ may be selected from the group consisting of H, phenyl, naphthyl, pyridyl, quinolinyl, CN, and CN-substituted phenyl. $R^1$ may be H and $R^2$ may be H or phenyl. $R^1$ may be H and $R^2$ may be phenyl. $R^1$ and $R^2$ may both be H.

In formula (II), L is selected from formulas (a) and (h) to (1)

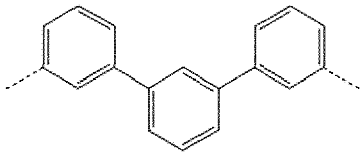
(a)
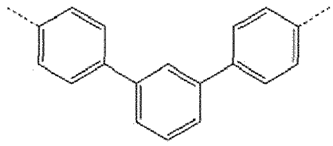
(h)
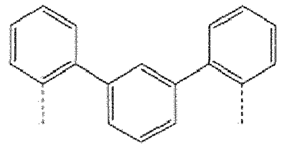
(i)
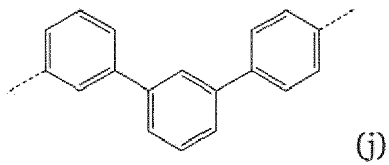
(j)
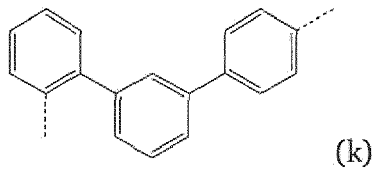
(k)
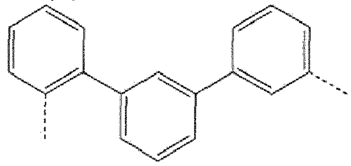
(l)

In the formulas (a) to (q), the dotted lines "------" represent the binding positions to

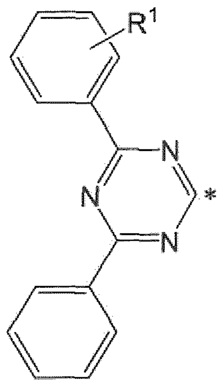
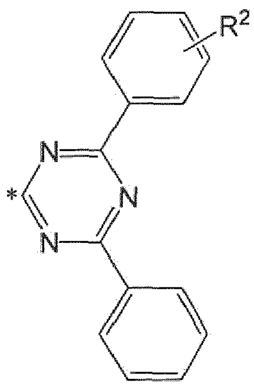
;

at the positions labeled with the *, respectively.

For example, if L has the formula (j), the compound of formula (II) has the formula

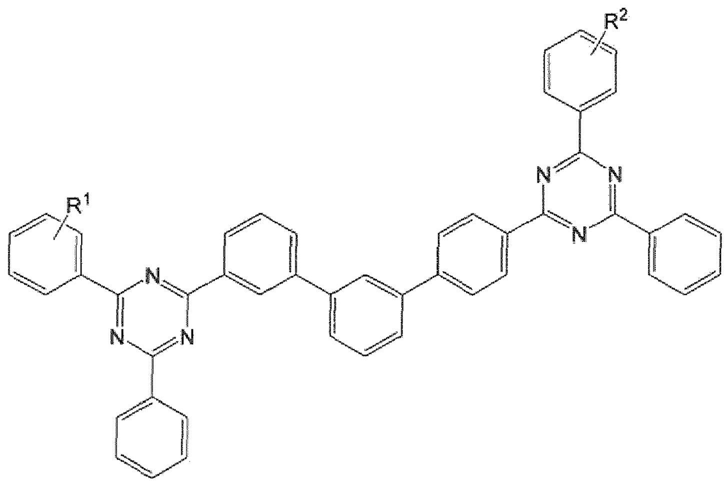

or

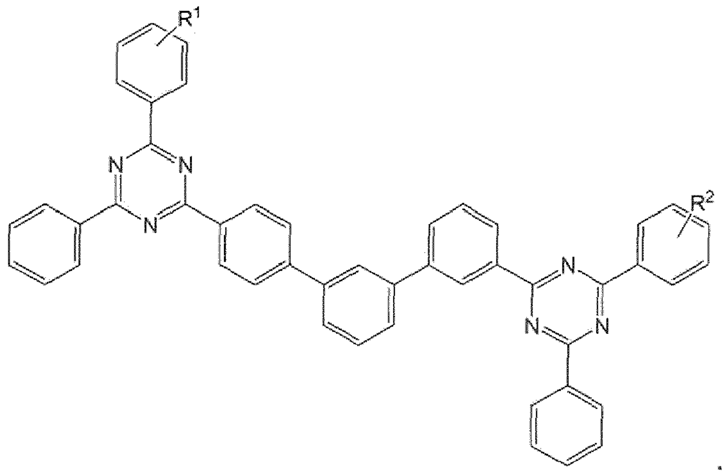

L may be (a)

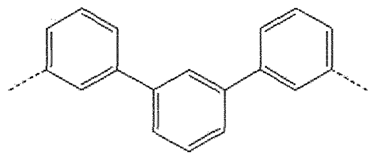

(a).

In one embodiment, there is provided an organic light emitting device comprising an anode, a cathode, an electron transport layer, an emission layer, and an auxiliary electron transport layer,
wherein

- the electron transport layer and the auxiliary electron transport layer are arranged between emission layer and the cathode;

- the auxiliary electron transport layer is arranged between the electron transport layer and the emission layer;

- the electron transport layer comprises a compound of formula (II)

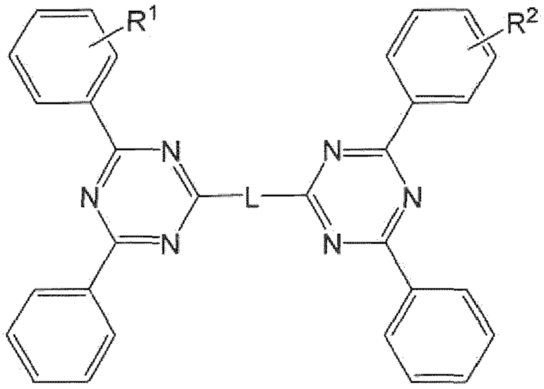

(II)

- $R^1$ and $R^2$ are independently selected from the group consisting of H, substituted or unsubstituted phenyl, substituted or unsubstituted naphthyl, substituted or unsubstituted pyridyl, substituted or unsubstituted quinolinyl, CN, and CN-substituted phenyl;
  - wherein if one or more of $R^1$ and $R^2$ is substituted $C_6$ to $C_{18}$ aryl or $C_3$ to $C_{17}$ heteroaryl, the one or more substituent(s) are independently selected from the group consisting of D and $C_1$ to $C_4$ alkyl;
- L is selected from formulas (a) and (h) to (l)

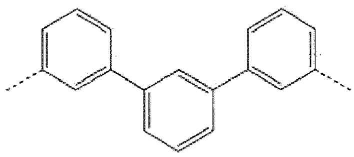

(a)

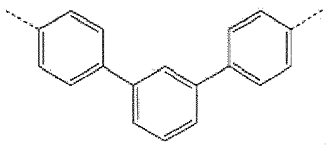

(h)

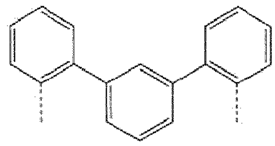

(i)

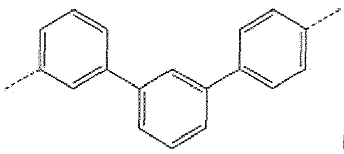

(j)

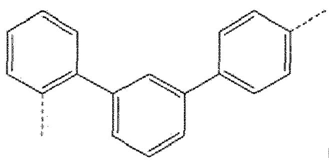

(k)

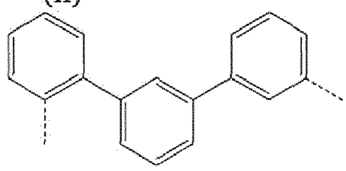

(l)

and

- the electron transport layer further comprises a lithium metal complex.

In one embodiment, there is provided an organic light emitting device comprising an anode, a cathode, an electron transport layer, an emission layer, and an auxiliary electron transport layer,
wherein

- the electron transport layer and the auxiliary electron transport layer are arranged between emission layer and the cathode;
- the auxiliary electron transport layer is arranged between the electron transport layer and the emission layer;
- the electron transport layer comprises a compound of formula (II)

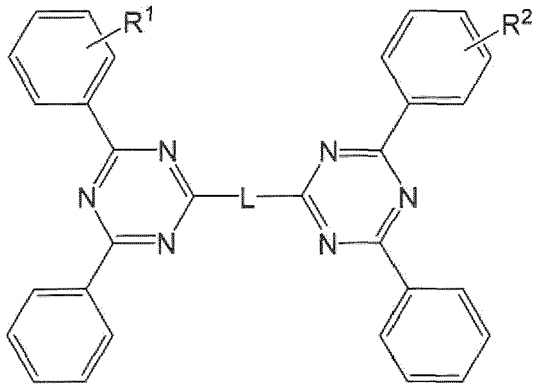

(II)

- $R^1$ and $R^2$ are independently selected from the group consisting of H, phenyl, naphthyl, pyridyl, quinolinyl, CN, and CN-substituted phenyl;
- L is (a)

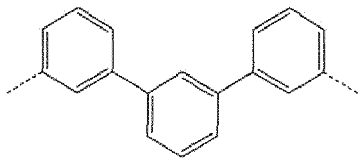

(a)

and

- the electron transport layer further comprises 8-hydroxyquinolinolato-lithium.

The compound of formula II may be selected from I-1 and I-12 to I-18

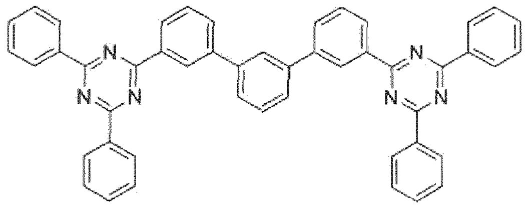

(I-1)

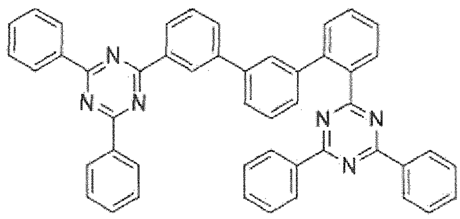

(I-12)

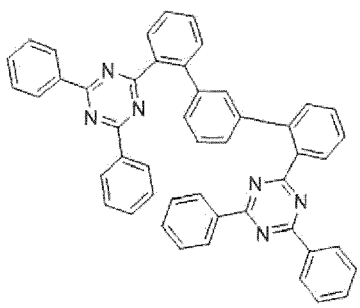

(I-13)

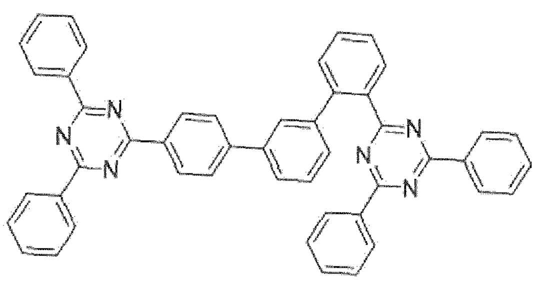

(I-14)

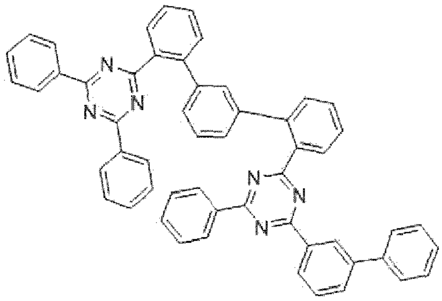
(I-15)

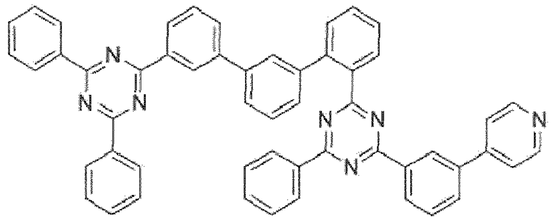
(I-16)

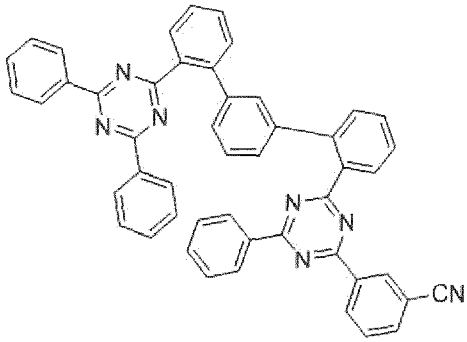
(I-17)

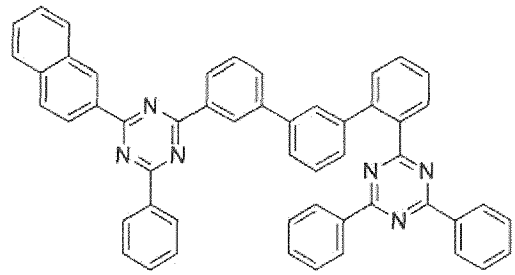
(I-18).

The compound of formula II may be I-1

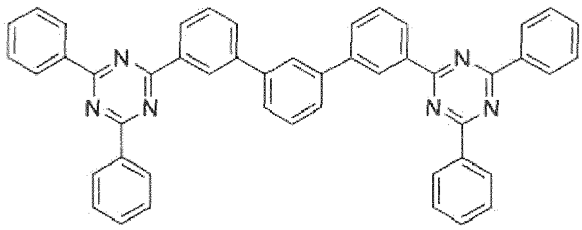
(I-1).

According to one embodiment of the organic light emitting device according to the invention, there is provided an organic light emitting device comprising an anode, a cathode, an electron transport layer, an emission layer, and an auxiliary electron transport layer,
wherein

- the electron transport layer and the auxiliary electron transport layer are arranged between emission layer and the cathode;
- the auxiliary electron transport layer is arranged between the electron transport layer and the emission layer;
- the electron transport layer comprises a compound of formula (III)

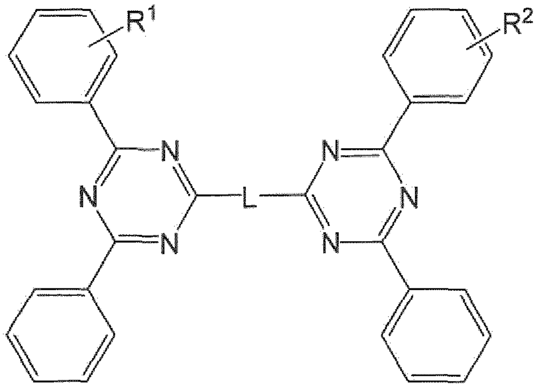

(III)

- $R^1$ and $R^2$ are independently selected from the group consisting of H, substituted or unsubstituted $C_6$ to $C_{18}$ aryl; substituted or unsubstituted $C_3$ to $C_{17}$ heteroaryl, CN, and CN-substituted $C_6$ to $C_{18}$ aryl;
  - wherein if one or more of $R^1$ and $R^2$ is substituted $C_6$ to $C_{18}$ aryl or $C_3$ to $C_7$ heteroaryl, the one or more substituent(s) are independently selected from the group consisting of D and $C_1$ to $C_4$ alkyl;
- L is selected from formulas (b) to (g)

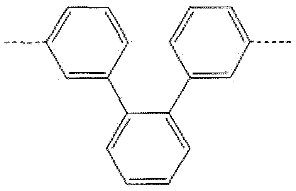

(b)

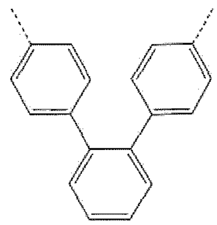

(c)

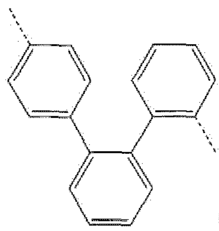

(d)

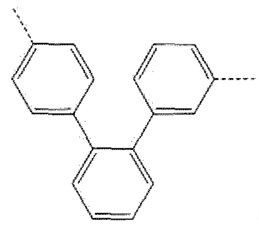

(e)

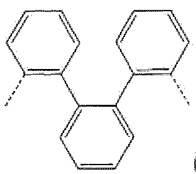

(f)

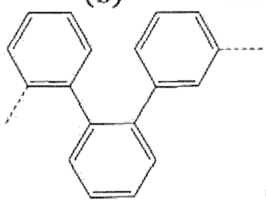

(g);

and

- the electron transport layer further comprises an alkali metal complex.

This embodiment encompasses layer structures, device architectures etc. described herein, especially described with respect to devices comprising the compound of formula (I), wherein the compound of formula (I) is replaced by the compound of formula (III).

The organic light emitting device comprises in an electron transport layer thereof a compound of Formula (III).

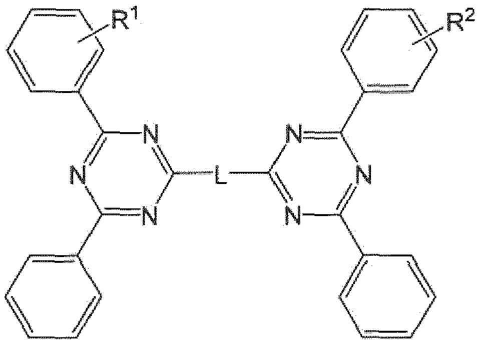

(III).

Especially, if not mentioned else explicitly, all compounds, especially the compound of formula (III), groups, moieties, substituents etc. shown herein, especially by structural formulas, by systematic names etc. encompass the respective partially and fully deuterated derivatives thereof, wherein it may be provided that substitution beyond deuteration is excluded.

In accordance with the present disclosure, as an illustrative example using an arbitrary group A in a formula showing the following binding situation,

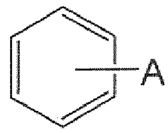

the group A may be bound to any suitable binding position. That is, in formula (III) $R^1$ may bonded to the positions marked with the *-symbol in the following formula, respectively

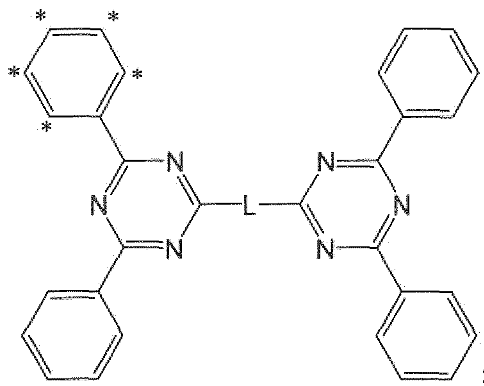

;

and in formula (III) $R^2$ may bonded to the positions marked with the *-symbol in the following formula, respectively

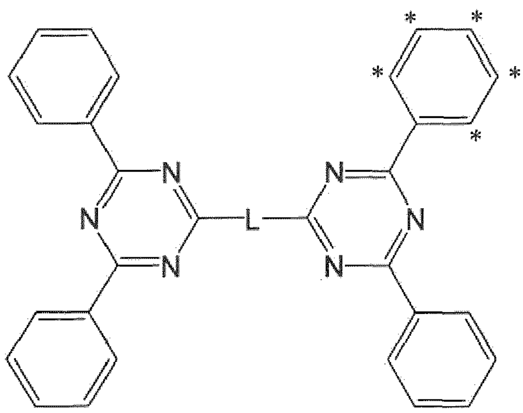

.

It is provided that the compound of formula (III) comprises exactly one group $R^1$ and exactly one group $R^2$. If one or both of $R^1$ and $R^2$ are H, the remaining H bonded to the respective phenyl ring are not considered as $R^1$, respectively $R^2$. In other words, the fact that in case that $R^1$ and/or $R^2$ is/are H more than one H is attached to the respective phenyl ring is not in conflict with the provision that the compound of formula (III) comprises exactly one group $R^1$ and exactly one group $R^2$ since only one of the H is considered as $R^1$/$R^2$.

In formula (III), $R^1$ and $R^2$ are independently selected from the group consisting of H, substituted or unsubstituted $C_6$ to $C_{18}$ aryl; substituted or unsubstituted $C_3$ to $C_{17}$ heteroaryl, CN, and CN-substituted $C_6$ to $C_{18}$ aryl. $R^1$ and $R^2$ may be independently selected from the group consisting of H, substituted or unsubstituted $C_6$ to $C_{12}$ aryl; substituted or unsubstituted $C_3$ to $C_{11}$ heteroaryl, CN, and CN-substituted $C_6$ to $C_{12}$ aryl. $R^1$ and $R^2$ may be independently selected from the group consisting of H, substituted or unsubstituted $C_6$ to $C_{10}$ aryl; substituted or unsubstituted $C_3$ to $C_9$ heteroaryl, CN, and CN-substituted $C_6$ to $C_{10}$ aryl. $R^1$ and $R^2$ may be independently selected from the group consisting of H, substituted or unsubstituted phenyl, substituted or unsubstituted naphthyl, substituted or unsubstituted pyridyl, substituted or unsubstituted quinolinyl, CN, and CN-substituted phenyl. $R^1$ and $R^2$ may be independently selected from the

group consisting of H and substituted or unsubstituted phenyl. $R^1$ and $R^2$ may both be H.

In formula (III), $R^1$ may be H and $R^2$ may be selected from the group consisting of H, substituted or unsubstituted $C_6$ to $C_{18}$ aryl; substituted or unsubstituted $C_3$ to $C_{17}$ heteroaryl, CN, and CN-substituted $C_6$ to $C_{18}$ aryl. $R^1$ may be H and $R^2$ may be selected from the group consisting of H, substituted or unsubstituted $C_6$ to $C_{12}$ aryl; substituted or unsubstituted $C_3$ to $C_{11}$ heteroaryl, CN, and CN-substituted $C_6$ to $C_{12}$ aryl. $R^1$ may be H and $R^2$ may be selected from the group consisting of H, substituted or unsubstituted $C_6$ to $C_{10}$ aryl; substituted or unsubstituted $C_3$ to $C_9$ heteroaryl, CN, and CN-substituted $C_6$ to $C_{10}$ aryl. $R^1$ may be H and $R^2$ may be selected from the group consisting of H, substituted or unsubstituted phenyl, substituted or unsubstituted naphthyl, substituted or unsubstituted pyridyl, substituted or unsubstituted quinolinyl, CN, and CN-substituted phenyl. $R^1$ may be H and $R^2$ may be H or substituted or unsubstituted phenyl. $R^1$ may be H and $R^2$ may be substituted or unsubstituted phenyl. $R^1$ and $R^2$ may both be H.

In formula (III), if one or more of $R^1$ and $R^2$ is substituted $C_6$ to $C_{18}$ aryl or $C_3$ to $C_{17}$ heteroaryl, the one or more substituent(s) are independently selected from the group consisting of D and $C_1$ to $C_4$ alkyl. If one or more of $R_1$ and $R^2$ is substituted $C_6$ to $C_{18}$ aryl or $C_3$ to $C_{17}$ heteroaryl, the one or more substituent(s) may be independently selected from the group consisting of D and $C_1$ to $C_2$ alkyl. If one or more of $R^1$ and $R^2$ is substituted $C_6$ to $C_{18}$ aryl or $C_3$ to $C_{17}$ heteroaryl, the one or more substituent(s) may be independently selected from the group consisting of D and methyl. If one or more of $R^1$ and $R^2$ is substituted $C_6$ to $C_{18}$ aryl or $C_3$ to $C_{17}$ heteroaryl, the one or more substituent(s) may be each D (deuterium).

In formula (III), $R^1$ and $R^2$ may be independently selected from the group consisting of H, $C_6$ to $C_{18}$ aryl; $C_3$ to $C_{17}$ heteroaryl, CN, and CN-substituted $C_6$ to $C_{18}$ aryl. $R^1$ and $R^2$ may be independently selected from the group consisting of H, $C_6$ to $C_{12}$ aryl; $C_3$ to $C_{11}$ heteroaryl, CN, and CN-substituted $C_6$ to $C_{18}$ aryl. $R^1$ and $R^2$ may be independently selected from the group consisting of H, $C_6$ to $C_{10}$ aryl; $C_3$ to $C_9$ heteroaryl, CN, and CN-substituted $C_6$ to $C_{10}$ aryl. $R^1$ and $R^2$ may be independently selected from the group consisting of H, phenyl, naphthyl, pyridyl, quinolinyl, CN, and CN-substituted phenyl. $R^1$ and $R^2$ may be independently selected from the group consisting of H and phenyl. $R^1$ and $R^2$ may both be H.

In formula (III), $R^1$ may be H and $R^2$ may be selected from the group consisting of H, $C_6$ to $C_{18}$ aryl; $C_3$ to $C_{17}$ heteroaryl, CN, and CN-substituted $C_6$ to $C_{18}$ aryl. $R^1$ may be H and $R^2$ may be selected from the group consisting of H, $C_6$ to $C_{12}$ aryl; $C_3$ to $C_{11}$, heteroaryl, CN, and CN-substituted $C_6$ to $C_{12}$ aryl. $R^1$ may be H and $R^2$ may be selected from the group consisting of H, $C_6$ to $C_{10}$ aryl; $C_3$ to $C_9$ heteroaryl, CN, and CN-substituted $C_6$ to $C_{10}$ aryl. $R^1$ may be H and $R^2$ may be selected from the group consisting of H, phenyl, naphthyl, pyridyl, quinolinyl, CN, and CN-substituted phenyl. $R^1$ may be H and $R^2$ may be H or phenyl. $R^1$ may be H and $R^2$ may be phenyl. $R^1$ and $R^2$ may both be H.

In formula (III), L is selected from formulas (a) to (g)

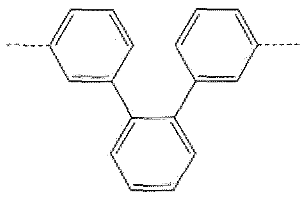
(b)

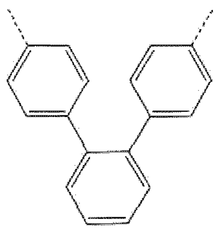
(c)

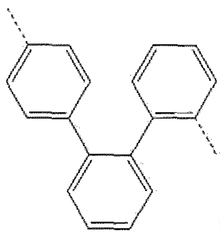
(d)

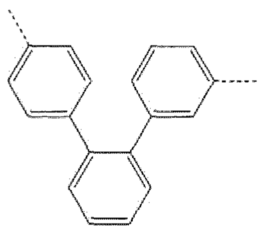
(e)

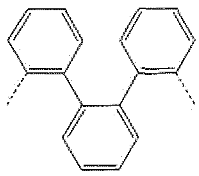
(f)

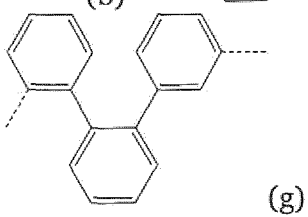
(g)

In the formulas (a) to (q), the dotted lines " - - - - - - " represent the binding positions to

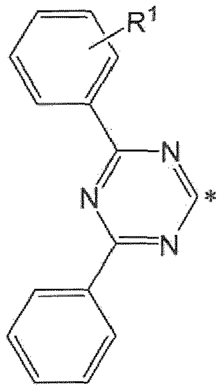 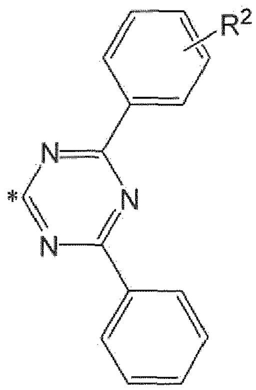

;

at the positions labeled with the *, respectively.

For example, if L has the formula (e), the compound of formula (III) has the formula

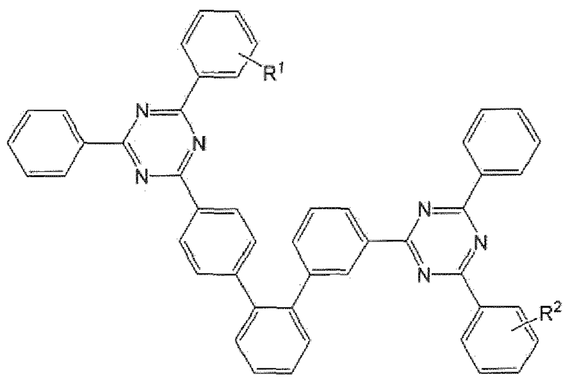

or

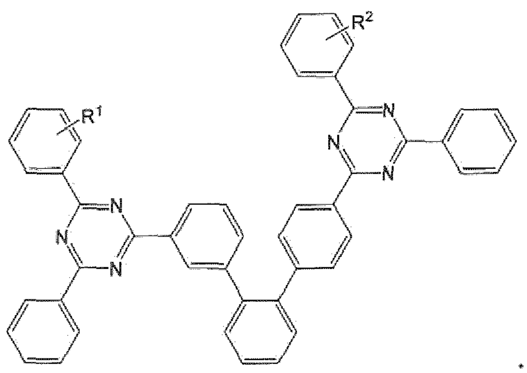

L may be selected from formulas (a) to (f)

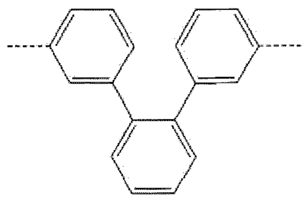 (b) 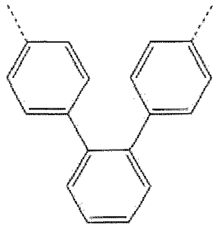 (c) 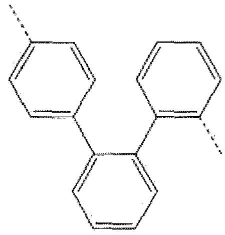 (d)

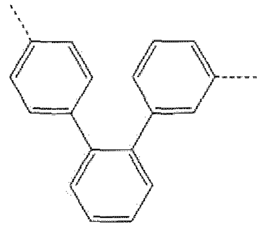 (e) 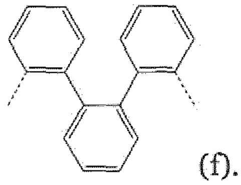 (f).

In one embodiment, there is provided an organic light emitting device comprising an anode, a cathode, an electron transport layer, an emission layer, and an auxiliary electron transport layer,
wherein

- the electron transport layer and the auxiliary electron transport layer are arranged between emission layer and the cathode;

- the auxiliary electron transport layer is arranged between the electron transport layer and the emission layer;

- the electron transport layer comprises a compound of formula (III)

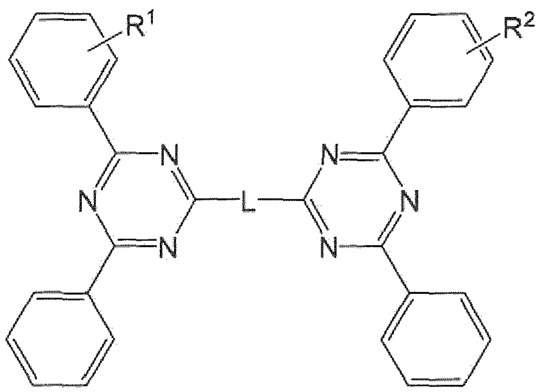 (III)

- $R^1$ and $R^2$ are independently selected from the group consisting of H, substituted or unsubstituted phenyl, substituted or unsubstituted naphthyl, substituted or unsubstituted pyridyl, substituted or unsubstituted quinolinyl, CN, and CN-substituted phenyl;

    - wherein if one or more of $R^1$ and $R^2$ is substituted $C_6$ to $C_{18}$ aryl or $C_3$ to $C_{17}$ heteroaryl, the one or more substituent(s) are independently selected from the group consisting of D and $C_1$ to $C_4$ alkyl;

- L is selected from formulas (b) to (g)

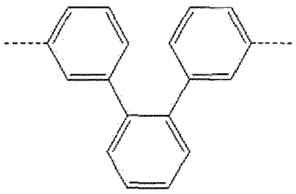
(b)
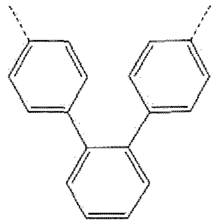
(c)
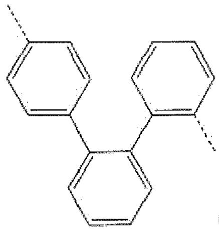
(d)
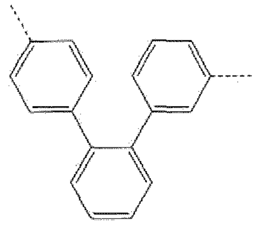
(e)
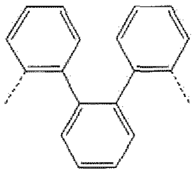
(f)
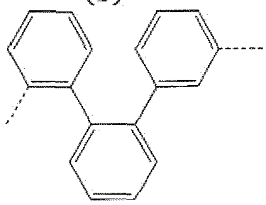
(g)

and

- the electron transport layer further comprises a lithium metal complex.

In one embodiment, there is provided an organic light emitting device comprising an anode, a cathode, an electron transport layer, an emission layer, and an auxiliary electron transport layer,
wherein

- the electron transport layer and the auxiliary electron transport layer are arranged between emission layer and the cathode;
- the auxiliary electron transport layer is arranged between the electron transport layer and the emission layer;
- the electron transport layer comprises a compound of formula (III)

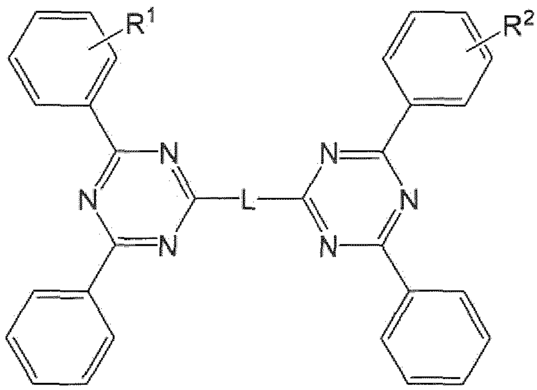
(III)

- $R^1$ and $R^2$ are independently selected from the group consisting of H, phenyl, naphthyl, pyridyl, quinolinyl, CN, and CN-substituted phenyl;
- L is selected from formulas (b) to (g)

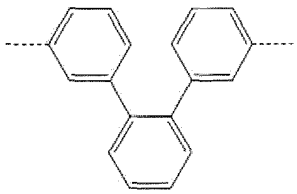 (b)
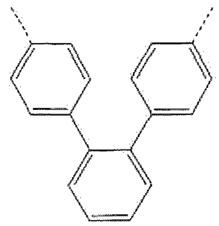 (c)
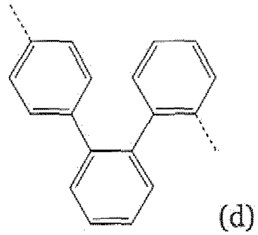 (d)
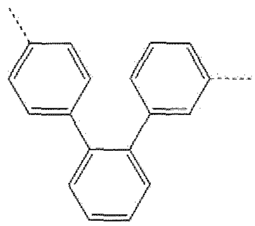 (e)
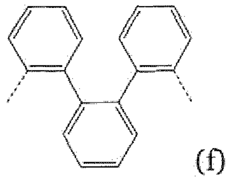 (f);

and

- the electron transport layer further comprises 8-hydroxyquinolinolato-lithium.

The compound of formula III may be selected from I-2 to I-11

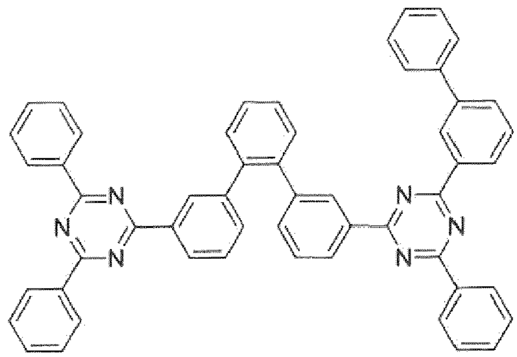 (I-2);

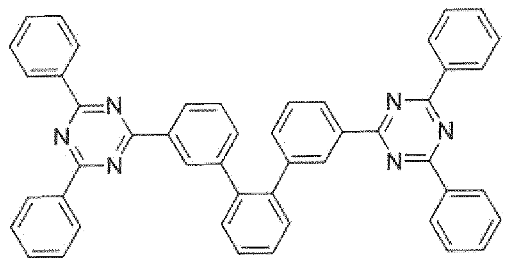 (I-3);

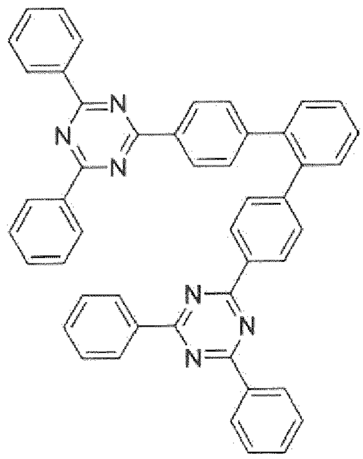

(I-4);

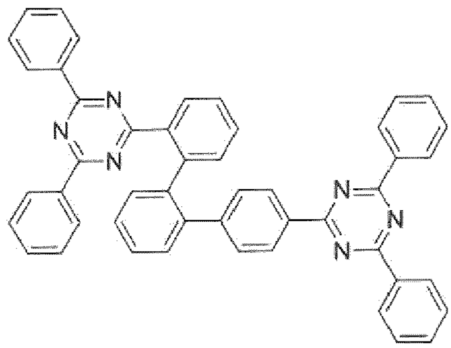

(I-5);

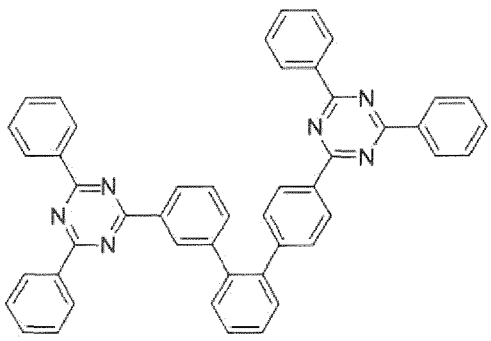

(I-6);

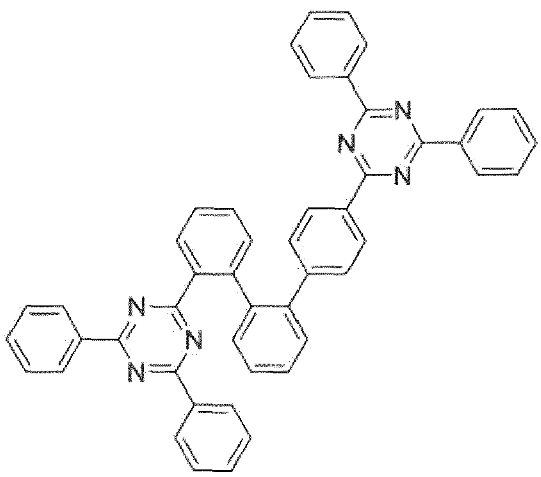

(I-7)

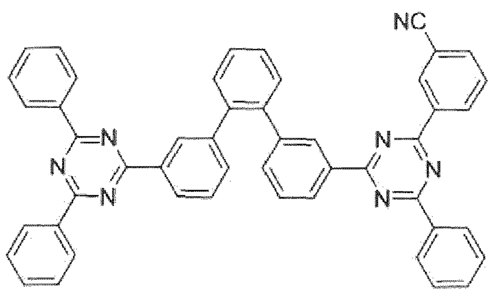

(I-8)

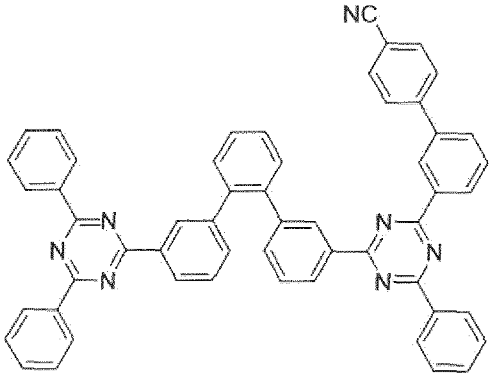

(I-9)

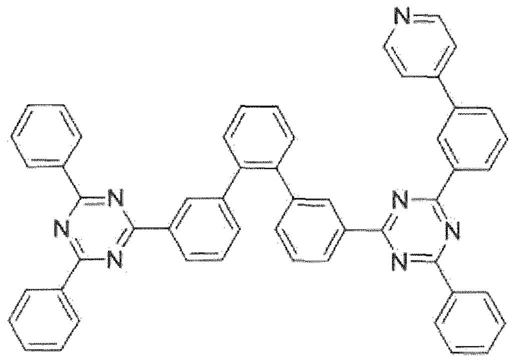

(I-10)

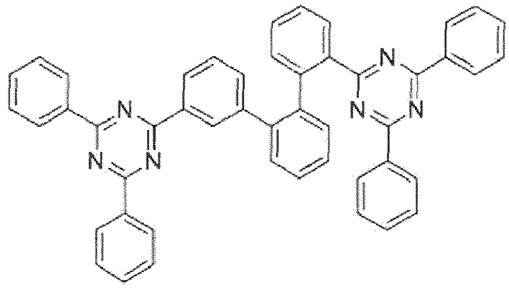

(I-11).

The compound of formula III may selected from I-2 to I-6

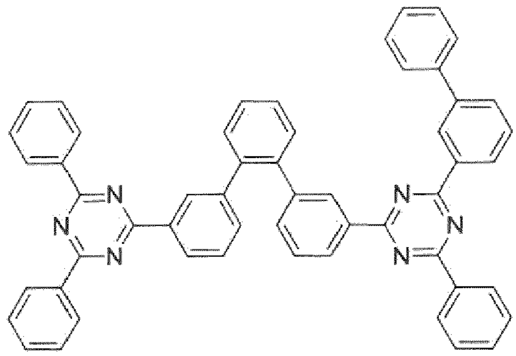

(I-2);

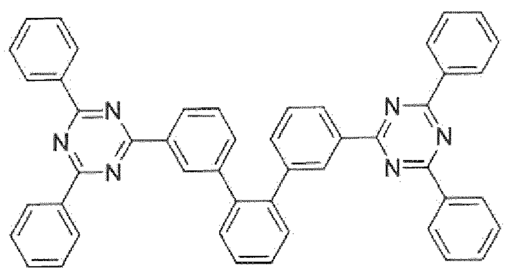

(I-3);

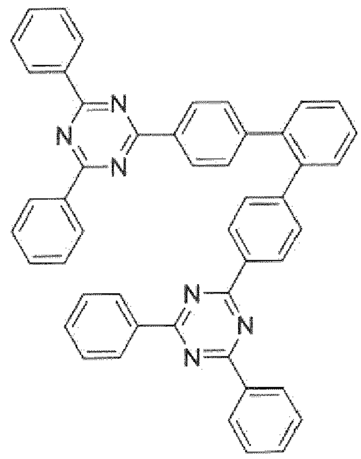

(I-4);

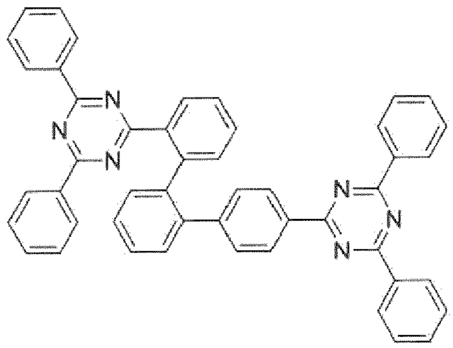

(I-5);

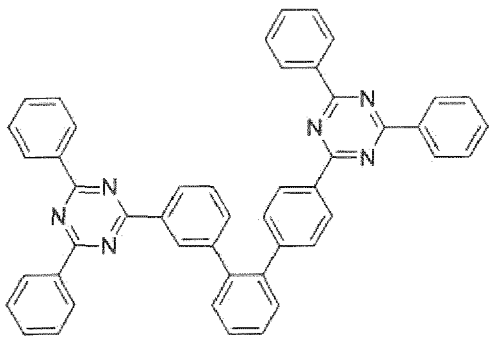

(I-6).

Alkali metal complex

The electron transport layer further comprises an alkali metal complex. The alkali metal complex may be an alkali metal organic complex.

The alkali metal complex may be a lithium complex. The alkali metal complex may be a lithium organic complex. The alkali metal complex may be a lithium chelate.

The alkali metal complex may be selected from lithium quinolinolate, lithium borate, lithium phenolate, lithium pyridinolate or from a lithium complex with a Schiff base ligand. The borate based organic ligand may be a tetra(1H-pyrazol-1-yl)borate. The phenolate may be a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate. The pyridinolate may be a 2-(diphenylphosphoryl)pyridin-3-olate. The lithium Schiff base has the structure 100, 101, 102 or 103

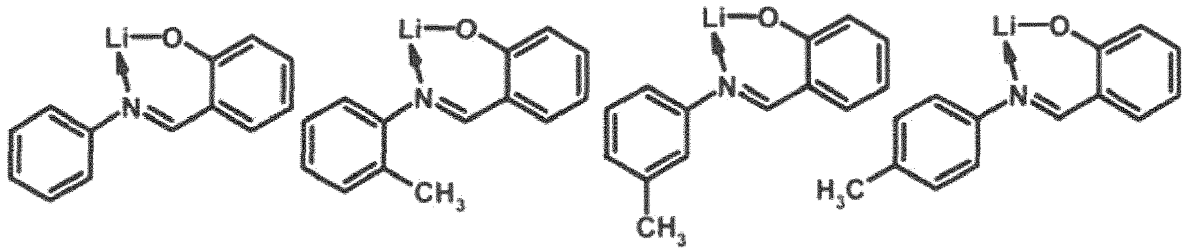

100 101 102 103

The alkali metal complex may have the formula II, III or IV:

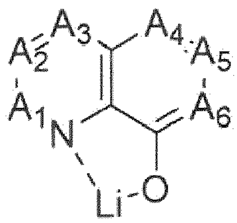 (II), 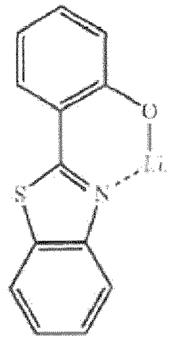 (III), 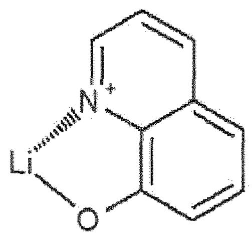 (IV)

wherein

$A_1$ to $A_6$ are same or independently selected from CH, CR, N, O;

R is same or independently selected from hydrogen, halogen, alkyl or aryl or heteroaryl with 1 to 20 carbon atoms; and more preferred $A_1$ to $A_6$ are CH.

The alkali metal complex may 8-hydroxyquinolinolato-lithium (= LiQ).

<u>Organic light emitting device</u>

The organic light emitting device in accordance with the invention comprises an anode, a cathode, an electron transport layer (which comprises the compound of formula (I)), an emission layer, and an auxiliary electron transport layer.

The organic light emitting device may be an organic light emitting diode. The organic light emitting diode is a single stack organic light emitting diode or a multi-stack organic light emitting diode.

The electron transport layer may comprise the compound of formula (I) in accordance with the invention in an amount of at least 50 wt.-% with respect to the total weight of the electron transport layer. The electron transport layer may comprise the compound of formula (I)in accordance with the invention in an amount of at least 60 wt.-% with respect to the total weight of the electron transport layer. The electron transport layer may comprise the compound of formula (I) in accordance with the invention in an amount of at least 70 wt.-% with respect to the total weight of the electron transport layer. The electron transport layer may comprise the compound of formula (I) in accordance with the invention in an amount of at least 80 wt.-% with respect to the total weight of the electron transport layer. The electron transport layer may comprise the compound of formula (I) in accordance with the invention in an amount of at least 90 wt.-% with respect to the total weight of the electron transport layer. The electron transport layer may comprise the compound of formula (I) in accordance with the invention in an amount of at least 95 wt.-% with respect to the total weight of the electron transport layer. The electron transport layer may comprise the compound of formula (I) in accordance with the invention in an amount of at least 98 wt.-% with respect to the total weight of the electron transport layer. The electron transport layer may comprise the compound of formula (I) in accordance with the invention in an amount of at least 99 wt.-% with respect to the total weight of the electron transport layer.

The electron transport layer may have a thickness from 10 to 50 nm, from 20 to 40 nm, or from 25 to 35 nm, such as about 30 nm, for example 31 nm.

The electron transport layer and the auxiliary electron transport layer are arranged between emission layer and the cathode. The auxiliary electron transport layer is arranged between the electron transport layer and the emission layer.

The auxiliary electron transport layer may be in direct contact with the electron transport layer. The auxiliary electron transport layer may be in direct contact with the emission layer. The auxiliary electron transport layer may be contacting sandwiched between the emission layer and the electron transport layer.

*Auxiliary electron transport layer*

The organic light emitting device comprises an auxiliary electron transport layer, wherein the auxiliary electron transport layer is arranged between the emission layer and the electron transport layer.

The auxiliary electron transport layer may also be referred to as a second electron transport layer or as a hole blocking layer.

The auxiliary electron transport layer may have a thickness of < 50 nm, optionally between 1 and 30 nm, optionally between 1 and 10 nm, optionally between 1 and 5 nm.

The auxiliary electron transport layer may comprise or consist of an auxiliary electron transport layer compound, wherein the auxiliary electron transport layer compound comprises 8 to 13 aromatic or heteroaromatic rings, optionally 8 to 11 aromatic or heteroaromatic rings, optionally 8 to 10 aromatic or heteroaromatic rings, and optionally 8 aromatic or heteroaromatic rings, wherein one or more of the aromatic or heteroaromatic rings may be substituted with $C_1$ to $C_4$ alkyl. In this regard, an aromatic, respectively heteroaromatic ring is a single aromatic ring, for example a 6-membered aromatic ring such as phenyl, a 6-membered heteroaromatic ring such as pyridyl, a 5-membered heteroaromatic ring such as pyrrolyl etc. In a system of condensed (hetero)aromatic rings, each ring is considered as a single ring in this regard. For example, naphthalene comprises two aromatic rings.

The auxiliary electron transport layer compound may comprise at least one heteroaromatic ring, optionally 1 to 5 heteroaromatic rings, optionally 1 to 4 heteroaromatic rings, optionally 1 to 3 heteroaromatic rings, and optionally 1 or 2 heteroaromatic rings.

The aromatic or heteroaromatic rings of the auxiliary electron transport layer compound may be 6-membered rings.

The heteroaromatic rings of the auxiliary electron transport layer compound may be a N-containing heteroaromatic ring, optionally all of the heteroaromatic rings are N-containing heteroaromatic rings, optionally all of the heteroaromatic rings heteroaromatic rings contain N as the only type of heteroatom.

The auxiliary electron transport layer compound may comprise at least one 6-membered heteroaromatic ring containing one to three N-atoms in each heteroaromatic ring, optionally one to three 6-membered heteroaromatic rings containing one to three N-atoms in each heteroaromatic ring, respectively.

The at least one 6-membered heteroaromatic ring comprised in the auxiliary electron transport layer compound may be an azine. The at least one 6-membered heteroaromatic ring comprised in the auxiliary electron transport layer compound may be triazine, diazine, such as pyrimidine, or pyrazine.

If the auxiliary electron transport layer compound comprises two or more heteroaromatic rings, the heteroaromatic rings may be separated from each other by at least one aromatic ring which is free of a heteroatom.

For example, the auxiliary electron transport layer may comprise or consist of the following compound

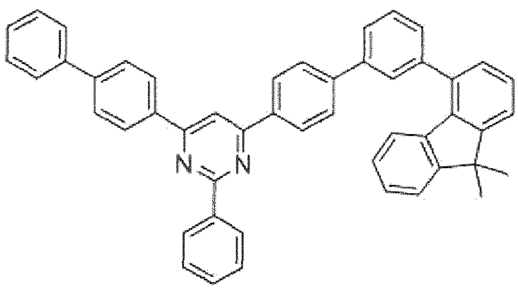

It may be provided that the auxiliary electron transport layer does not comprise the compound of formula (I).

*Electron injection layer*

The organic light emitting device may further comprise an electron injection layer, wherein the electron injection layer is arranged between the electron transport layer and the cathode. The electron injection layer may be in direct contact with the electron transport layer. The electron injection layer may be in direct contact with the cathode. The electron injection layer may be contacting sandwiched between the electron transport layer and the cathode.

The electron transport layer may be contacting sandwiched between the auxiliary electron transport layer and the

electron injection layer.

It may be provided that the electron injection layer does not comprise the compound of formula (I).

The electron injection layer may comprise a metal salt or a metal complex, preferably a lithium salt or a lithium organic complex; more preferably a compound selected from lithium halides and lithium organic chelates; even more preferably from lithium fluoride, a lithium quinolinolate, lithium borate, lithium phenolate, lithium pyridinolate or from a lithium complex with a Schiff base ligand; most preferably,

- the lithium complex has the formula II, III or IV:

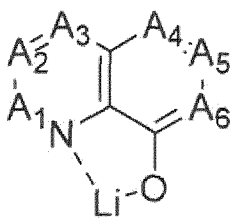 (II), 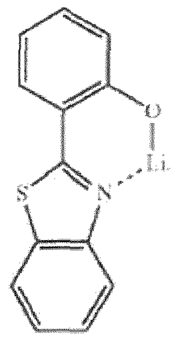 (III), 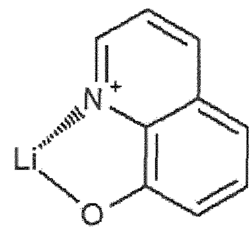 (IV)

wherein

$A_1$ to $A_6$ are same or independently selected from CH, CR, N, O;

R is same or independently selected from hydrogen, halogen, alkyl or aryl or heteroaryl with 1 to 20 carbon atoms; and more preferred $A_1$ to $A_6$ are CH,

- the borate based organic ligand is a tetra(1H-pyrazol-1-yl)borate,

- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,

- the pyridinolate is a 2-(diphenylphosphoryl)pyridin-3-olate,

- the lithium Schiff base has the structure 100, 101, 102 or 103:

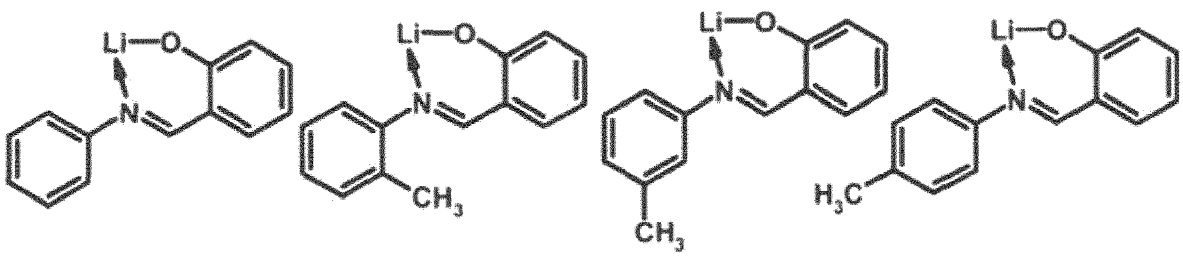

**100** **101** **102** **103**

The electron injection layer may comprise or consist of a metal selected form the group consisting of alkali metals, alkaline earth metals, and rare earth metals, preferably the metal may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb most preferred Yb.

The thickness of the electron injection layer may be in the range from about 0.1 nm to about 10 nm, for example, in the range from about 0.5 nm to about 5 nm. When the thickness of the EIL is within this range, the EIL may have satisfactory electron-injecting properties, without a substantial penalty in driving voltage.

*Further layers*

In accordance with the invention, the organic light emitting device may comprise, besides the layers already mentioned above, further layers. Exemplary embodiments of respective layers are described in the following:

*Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate. It may be provided that the substrate is a non-transparent substrate.

*Anode electrode*

The inventive organic light emitting device comprises an anode (anode electrode). The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide ($SnO_2$), aluminum zinc oxide (AlZO) and zinc oxide (ZnO), may be used to form the anode electrode. The anode electrode may also be formed using metals, typically silver (Ag), gold (Au), or metal alloys.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane ($F_4TCNQ$), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane ($F_4TCNQ$), which LUMO level is about -5.2 eV; zinc phthalocyanine (ZnPc) (HOMO = -5.2 eV) doped with $F_4TCNQ$; α-NPD (N,N'-Bis(naphthalen-1-yl)-N,N'-bis(phenyl)-benzidine) doped with $F_4TCNQ$. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the

conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-i-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Emission layer (EML)*

The emission layer in the organic light emitting device in accordance with the invention may be a blue emission layer or a green emission layer.

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc ($Zn(BTZ)_2$).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, $Ir(piq)_3$, and $Btp_2Ir(acac)$, but are not limited thereto. These compounds are phosphorescent emitters, however, fluorescent red emitter dopants could also be used.

Examples of phosphorescent green emitter dopants are $Ir(ppy)_3$ (ppy = phenylpyridine), $Ir(ppy)_2(acac)$, $Ir(m\text{-}pyp)_3$.

Examples of phosphorescent blue emitter dopants are $F_2Irpic$, $(F_2ppy)_2Ir(tmd)$ and $Ir(dfppz)_3$ and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

The hole blocking layer may be the auxiliary electron transport layer as defined above. The hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

The hole blocking layer may also be described as a-ETL or auxiliary ETL.

The HBL (= a-ETL) may be free of the compound of formula (I).

*Electron transport layer (ETL)*

The OLED according to the present invention comprises one or more electron transport layer(s) (ETL). In accordance with the invention, at least one of the electron transport layers is the inventive electron transport layer comprising the compound of formula (I) as defined herein.

By suitably adjusting energy levels of particular layers of the ETL, the injection and transport of the electrons may be controlled, and the holes may be efficiently blocked. Thus, the OLED may have long lifetime.

The electron transport layer may comprise, besides the compound of formula (I) further ETM materials known in the art. Likewise, the electron transport layer may comprise as the only electron transport matrix material the compound of formula (I). In case that the inventive organic electronic device comprises more than one electron transport layers, the compound of formula (I) may be comprised in only one of the electron transport layers, in more than one of the electron transport layers or in all of the electron transport layers. Suitable further compounds for the ETM are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

*Electron injection layer (EIL)*

The electron injection layer may be as described above. An EIL, which may facilitate injection of electrons from the cathode into the electron transport layer stack, may be formed on the electron transport layer stack, preferably directly on the electron transport layer stack, preferably directly on the second electron transport layer, preferably in direct contact with the second electron transport layer. Examples of materials for forming the EIL or being comprised in the EIL include lithium 8-hydroxyquinolinolate (LiQ), LiF, NaCl, CsF, $Li_2O$, BaO, Ca, Ba, Yb, Mg which are known in the art. Deposition and coating conditions for forming the EIL are similar to those for formation of the HIL, although the deposition and coating conditions may vary, according to the material that is used to form the EIL. The EIL may comprise an organic matrix material doped with an n-type dopant. The matrix material may be selected from materials conventionally used as matrix materials for electron transport layers.

The EIL may consist of a number of individual EIL sublayers. In case the EIL consists of a number of individual EIL sublayers, the number of sublayers is preferably 2. The individual EIL sublayers may comprise different materials for forming the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

It may be provided that the electron injection layer does not comprise the compound of formula (I).

*Cathode electrode*

The cathode (cathode electrode) is formed on the EIL (if present) or on the ETL, preferably directly on the EIL, preferably in direct contact with the EIL. In the sense of this invention the cathode and the EIL can be regarded as one functional part enabling the injection of electrons into the electron transport layer stack. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like, for example an alloy of Ag and Mg, such as Ag:Mg 90:10 wt/wt. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy. The transparent or semitransparent cathode may facilitate light emission through the cathode.

*Charge generation layer (CGL)*

The charge generation layer (CGL) may comprise a p-type charge generation layer (p-CGL) and an n-type charge generation layer (n-CGL). An interlayer may be arranged between the p-CGL and the -n-CGL.

Typically, the charge generation layer is a p-n junction joining an n-type charge generation layer (electron generating layer) and a hole generating layer. The n-side of the p-n junction generates electrons and injects them into the layer that is adjacent in the direction to the anode. Analogously, the p-side of the p-n junction generates holes and injects them into the layer that is adjacent in the direction to the cathode.

Charge generating layers are used in tandem and stacked devices, for example, in tandem or stacked OLEDs comprising, between two electrodes, two or more emission layers. In a tandem or stacked OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluoro-7,7,8,8-tetracyanoquinodimethane ($F_4$-TCNQ), derivatives of tetracyanoquinodimethane, radialene derivatives, iodine, $FeCl_3$, $FeF_3$, and $SbCl_5$. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generating layer may be the layer comprising the compound of Formula (I). The n-type charge generation layer can be layer of a neat n-type dopant, for example of a metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Li, Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline) aluminum, benzazole derivatives, and silole derivatives.

According to one aspect of the present invention, an electron transport layer comprising the compound of formula (I) is arranged between a first and a second emission layer and a further electron transport layer comprising the compound of formula (I) is arranged between the second emission layer and the cathode.

According to another aspect of the present invention, there is provided an OLED comprising: a substrate; an anode electrode formed on the substrate; a hole injection layer, a hole transport layer, an electron blocking layer, an emission layer, an auxiliary electron transport layer, an electron transport layer comprising the compound of formula (I) and a cathode electrode.

According to another aspect of the present invention, there is provided an OLED comprising: a substrate; an anode electrode formed on the substrate; a hole injection layer, a hole transport layer, an electron blocking layer, an emission layer, a auxiliary electron transport layer layer, an electron transport layer comprising the compound of formula (I), an electron injection layer, and a cathode electrode.

According to various embodiments of the present invention, there may be provided OLEDs layers arranged between the above mentioned layers, on the substrate or on the top electrode.

In one embodiment, the organic light emitting device according to the invention further comprises a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

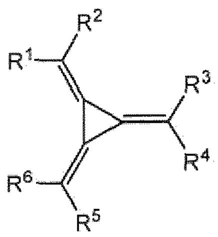
(XX)
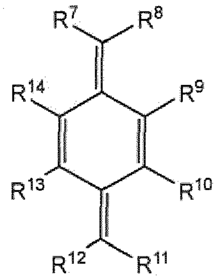
(XXIa)
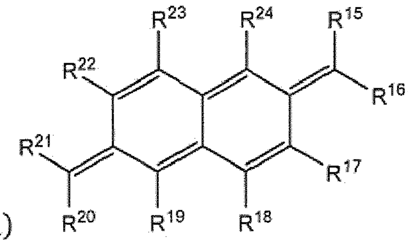
(XXIb),

wherein (as an exception different to the description above) $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

Process for preparing the organic light emitting device

According to a further aspect, the invention is related to a process for preparing the organic light emitting device according to the present invention, wherein the process comprises a step of depositing the compound of formula (I) according to the present invention on a solid support.

The method for depositing may comprise:

- deposition via vacuum thermal evaporation;
- deposition via solution processing, preferably the processing is selected from spin-coating, printing, casting; and/or
- slot-die coating.

Display device

According to a further aspect, the invention is related to a display device comprising the organic light emitting device according to the invention, preferably comprises at least two organic light emitting devices according to the invention.

The display device may be a television, a tablet, or a mobile phone.

GENERAL DEFINITIONS

If not explicitly mentioned else, each moiety of the compounds described herein, especially the compounds of formulas (I) may be substituted with one or more D (deuterium).

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, $C_3$-alkyl may be selected from n-propyl and iso-propyl. Likewise, $C_4$-alkyl encompasses n-butyl, sec-butyl and t-butyl. Likewise, $C_6$-alkyl encompasses n-hexyl and cyclo-hexyl.

As used herein if not explicitly mentioned else, the asterisk symbol "*" represents a binding position at which the moiety labelled accordingly is bond to another moiety.

The subscribed number n in $C_n$ relates to the total number of carbon atoms in the respective alkyl, arylene, heteroarylene or aryl group.

The term "aryl" or "arylene" as used herein shall encompass phenyl ($C_6$-aryl), fused aromatics, such as

naphthalene, anthracene, phenanthrene, tetracene etc.. Further encompassed are biphenyl and oligo- or polyphenyls, such as terphenyl, phenyl-substituted biphenyl, phenyl-substituted terphenyl (such as tetraphenyl benzene groups) etc.. "Arylene" respectively "heteroarylene", refers to groups to which two further moieties are attached. In the present specification, the term "aryl group" or "arylene group" may refer to a group comprising at least one hydrocarbon aromatic moiety, and all the elements of the hydrocarbon aromatic moiety may have p-orbitals which form conjugation, for example a phenyl group, a napthyl group, an anthracenyl group, a phenanthrenyl group, a pyrenyl group, a fluorenyl group and the like. Further encompoassed are spiro compounds in which two aromatic moieties are connected with each other via a spiro-atom, such as 9,9'-spirobi[9H-fluorene]yl. The aryl or arylene group may include a monocyclic or fused ring polycyclic (i.e., links sharing adjacent pairs of carbon atoms) functional group.

The term "heteroaryl" as used herein refers to aryl groups in which at least one carbon atom is substituted with a heteroatom. The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably, a heteroarylene ring may comprise at least 1 to 3 heteroatoms individually selected from N, 5 and/or O. Just as in case of "aryl"/"arylene", the term "heteroaryl" comprises, for example, spiro compounds in which two aromatic moieties are connected with each other, such as spiro[fluorene-9,9'-xanthene]. Further exemplary heteroaryl groups are diazine, triazine, dibenzofurane, dibenzothiofurane, acridine, benzoacridine, dibenzoacridine etc.

The term "alkenyl" as used herein refers to a group $-CR^1 = CR^2R^3$ comprising a carbon-carbon double bond.

The term "perhalogenated" as used herein refers to a hydrocarbyl group wherein all of the hydrogen atoms of the hydrocarbyl group are replaced by halogen (F, Cl, Br, I) atoms.

The term "alkoxy" as used herein refers to a structural fragment of the Formula -OR with R being hydrocarbyl, preferably alkyl or cycloalkyl.

The term "thioalkyl" as used herein refers to a structural fragment of the Formula -SR with R being hydrocarbyl, preferably alkyl or cycloalkyl.

The subscripted number n in $C_n$-heteroaryl merely refers to the number of carbon atoms excluding the number of heteroatoms. In this context, it is clear that a $C_3$ heteroarylene group is an aromatic compound comprising three carbon atoms, such as pyrazol, imidazole, oxazole, thiazole and the like.

The term "heteroaryl" as used herewith shall encompass pyridine, quinoline, benzoquinoline, quinazoline, benzoquinazoline, pyrimidine, pyrazine, triazine, benzimidazole, benzothiazole, benzo[4,5]thieno[3,2-d]pyrimidine, carbazole, xanthene, phenoxazine, benzoacridine, dibenzoacridine and the like.

In the present specification, the term single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In accordance with the present disclosure, as an illustrative example using an arbitrary group A in a formula showing the following binding situation,

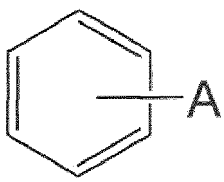

the group A may be bound to any suitable binding position. In a situation where it is shown that the bond of A crosses more than one ring

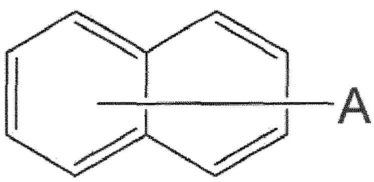

the group A may be bound to any suitable binding position of each ring crossed with the bond.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.
The term "contacting sandwiched" refers to an arrangement of three layers whereby the layer in the middle is in direct contact with the two adjacent layers.
With respect to the inventive electron transport layer stack the compounds mentioned in the experimental part are most preferred.
A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.
According to another aspect, the organic electroluminescent device according to the present invention comprises two or three or more emission layers. An OLED comprising more than one emission layer is also described as a tandem OLED or stacked OLED.
The organic electroluminescent device (OLED) may be a bottom- or top-emission device. The organic electroluminescent device (OLED) may emit the light through a transparent anode or through a transparent cathode.
Another aspect is directed to a device comprising at least one organic electroluminescent device (OLED).
A device comprising organic light-emitting diodes is for example a display or a lighting panel.
In the present invention, the following defined terms, these definitions shall be applied, unless a different definition is given in the claims or elsewhere in this specification.
In the context of the present specification the term "different" or "differs" in connection with the matrix material means that the matrix material differs in their structural Formula.
The terms "OLED" and "organic light-emitting diode" are used simultaneously and have the same meaning. The term "organic electroluminescent device" as used herein may comprise both organic light emitting diodes as well as organic light-emitting transistors (OLETs).
As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.
As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.
All numeric values are herein assumed to be modified by the term "about", whether or not explicitly indicated. As used herein, the term "about" refers to variation in the numerical quantity that can occur. Whether or not modified by the term "about" the claims include equivalents to the quantities.
It should be noted that, as used in this specification and the appended claims, the singular forms "a", "an", and "the" include plural referents unless the content clearly dictates otherwise.
The term "free of", "does not contain", "does not comprise" does not exclude impurities. Impurities have no technical effect with respect to the object achieved by the present invention.
In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.
Preferably, the electron transport layer comprising the compound of Formula (I) is essentially non-emissive or non-emitting.
The operating voltage, also named U, is measured in Volt (V) at 10 milliampere per square centimeter ($mA/cm^2$).
The candela per ampere efficiency, also named cd/A efficiency or $C_{eff}$, is measured in candela per ampere at 10 milliampere per square centimeter ($mA/cm^2$).
The external quantum efficiency, also named EQE, is measured in percent (%).
The color space is described by coordinates CIE-x and CIE-y (International Commission on Illumination 1931). For blue emission the CIE-y is of particular importance. A smaller CIE-y denotes a deeper blue color. Efficiency values are compared at the same CIE-y.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

The anode and cathode may be described as anode electrode / cathode electrode or anode electrode / cathode electrode or anode electrode layer / cathode electrode layer.

Room temperature, also named ambient temperature, is 23°C.

Hereinafter, the embodiments are illustrated in more detail with reference to examples. However, the present disclosure is not limited to the following examples. Reference will now be made in detail to the exemplary aspects.

DESCRIPTION OF THE DRAWINGS

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures, which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

FIG. 1 is a schematic sectional view of an OLED, according to an exemplary embodiment of the present invention.

FIG. 2 is a schematic sectional view of an OLED comprising a charge generation layer and two emission layers, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an electron blocking layer (EBL) 145, an emission layer (EML) 150, a auxiliary electron transport layer 155, and an electron transport layer (ETL) 160. The electron transport layer (ETL) 160 is formed on the aEML 155. The electron transport layer (ETL) 160 comprises the compound of formula (1) and an alkali metal complex. Onto the electron transport layer (ETL) 160, an electron injection layer (EIL) 180 is disposed. The cathode 190 is disposed directly onto the electron injection layer (EIL) 180.

Fig. 2 is a schematic sectional view of an OLED 100, according to another exemplary embodiment of the present invention. Fig. 2 differs from Fig. 1 in that the OLED 100 of Fig. 2 further comprises a charge generation layer (CGL) and a second emission layer (151).

Referring to Fig. 2, the OLED 100 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156 which is the auxiliary electron transport layer according to the invention, a second electron transport layer (ETL) 161 which is the electron transport layer which comprises the compound of formula (1) and an alkali metal complex according to the invention, a second electron injection layer (EIL) 181 and a cathode 190.

While not shown in Fig. 1 and Fig. 2 a sealing layer may further be formed on the cathode electrodes 190, in order to seal the OLEDs 100. In addition, various other modifications may be applied thereto.

Hereinafter, the embodiments are illustrated in more detail with reference to examples. However, the present disclosure is not limited to the following examples.

EXPERIMENTAL PART

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Rate onset temperature

The rate onset temperature ($T_{RO}$) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

Reduction potential

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard Fc+/Fc redox couple, afforded finally the values reported above. All studied compounds as well as the reported comparative compounds showed well-defined reversible electrochemical behaviour.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r}_i$ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

**Synthesis ProceduresCompound I-**2 - **2-([1,1'-biphenyl]-3-yl)-4-(3"-(4,6-diphenyl-1,3,5-triazin-2-yl)-[1,1':2',1"-terphenyl]-3-yl)-6-phenyl-1,3,5-triazine**

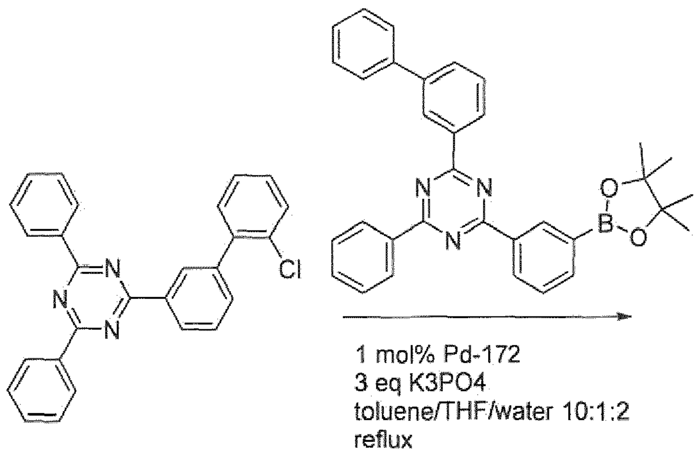


3 Neck round-bottom flask was flushed with nitrogen and charged with 2-(2'-chloro-[1,1'-biphenyl]-3-yl)-4,6-diphenyl-1,3,5-triazine (CAS 2649455-14-9) 1 eq (16,2 g), 2-([1,1'-biphenyl]-3-yl)-4-phenyl-6-(3-(4,4,5,5-tetramethyl-1,3,2-dioxaborolan-2-yl)phenyl)-1,3,5-triazine (CAS 2032365-30-1) 0,95 eq (20,8 g), chloro(crotyl)(2-dicyclohexylphosphino-2',6'-dimethoxybiphenyl)palladium(II) (CAS 1798781-99-3) 0,01 eq (0,3 g), potassium phosphate (CAS 7778-53-2) 3 eq (29,0 g). Deaerated mixture of 200 ml toluene, 20 ml THF and 40 ml water was added. Reaction was running at reflux under a nitrogen atmosphere for 3 days. Then the reaction mixture was cooled down, solvent was evaporated and the crude solid mixture was extracted in DCM, washed with water, dried over MgSO4. Then the material was purified by column chromatography using mixture of DCM/hexane 1:2 as a mobile phase. Final purification was done by sublimation. White powder. Yield: 16,2 g (56%). (ESI-MS: 769).

**Compound I-3 - 3,3"-bis(4,6-diphenyl-1,3,5-triazin-2-yl)-1,1':2',1"-terphenyl. CAS# 2055848-25-2**

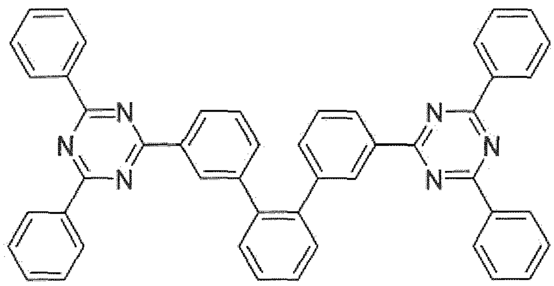

**Compound I-4 - 4,4"-bis(4,6-diphenyl-1,3,5-triazin-2-yl)-1,1':2',1"-terphenyl. CAS# 2230476-56-7**

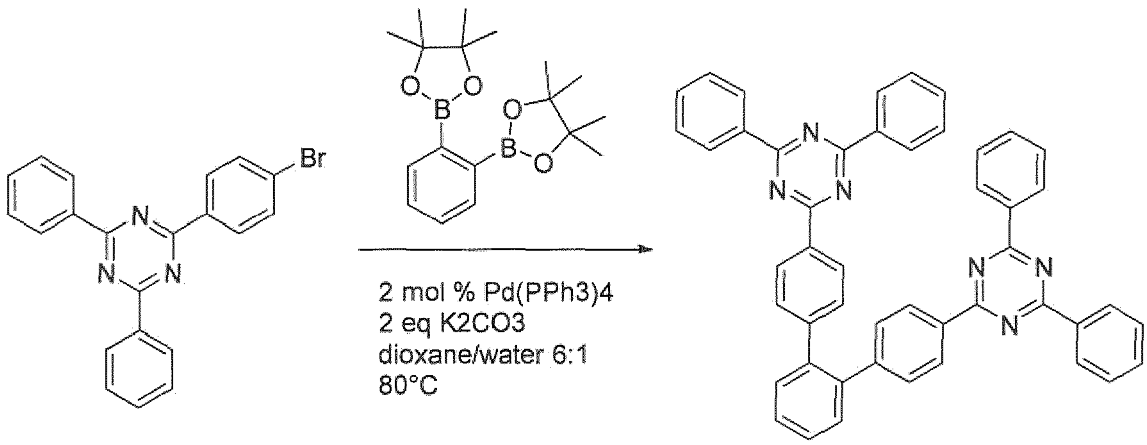


3 Neck round-bottom flask was flushed with nitrogen and charged with 1,2-bis(4,4,5,5-tetramethyl-1,3,2-dioxaborolan-2-yl)benzene (CAS 269410-07-3), 2-(4-bromophenyl)-4,6-diphenyl-1,3,5-triazine 1 eq (8,o g), 2-(4-bromophenyl)-4,6-diphenyl-1,3,5-triazine (CAS 23449-08-3) 2,1 eq (19,8 g), tetrakis(triphenylphosphine)palladium(0) (CAS 14221-01-3) 0,02 eq (0,6 g), potassium carbonate (CAS 584-08-7) 2 eq (6,7 g). Deaerated mixture of 150 ml dioxane and 25 ml water was added. Reaction was running at 80°C under a nitrogen atmosphere overnight. After cooling down to room temperature gray suspension was formed. Raw solid material was filtered, washed with water and methanol. Then the product was dissolved in 800 ml hot chlorobenzene and filtrated thextracted in Soxhlet apparatus with chlorobenzene. After cooling down the extract was partially evaporated, white precipitate was formed and filtered. Final purification was done by sublimation. White powder. Yield: 4,5 g (27%). (ESI-MS: 693).

**Compound 1-6 - 6,6'-([1,1':2',1"-terphenyl]-3,4"-diyl)bis(2,4-diphenyl-1,3,5-triazine)**

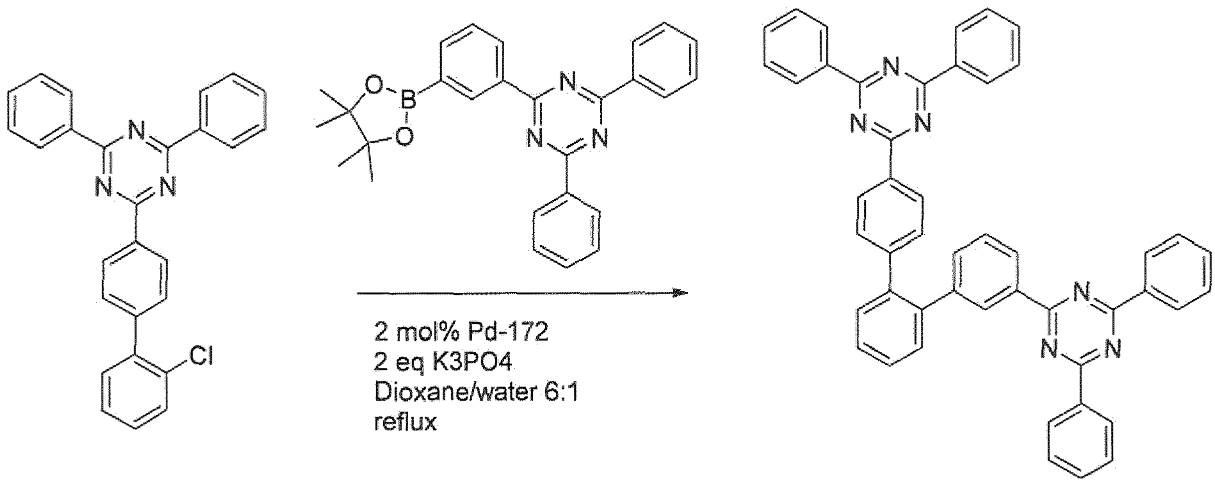


3 Neck round-bottom flask was flushed with nitrogen and charged with 2-(2'-chloro-[1,1'-biphenyl]-4-yl)-4,6-diphenyl-1,3,5-triazine (CAS 2243925-15-5) 1 eq (10,0 g), 2,4-diphenyl-6-(3-(4,4,5,5-tetramethyl-1,3,2-dioxaborolan-2-yl)phenyl)-1,3,5-triazine (CAS 1269508-31-7) 1 eq (10,4 g), chloro(crotyl)(2-dicyclohexylphosphino-2',6'-dimethoxybiphenyl)palladium(II) (CAS 1798781-99-3) 0,02 eq (0,3 g), potassium phosphate (CAS 7778-53-2) 2 eq (10,1 g). Deaerated mixture of 240 ml dioxane and 40 ml water was added. Reaction was running at reflux under a nitrogen atmosphere overnight. After cooling down to room temperature gray suspension was formed. Raw solid material was filtered, washed with water. Then the product was dissolved in 800 ml of hot chlorobenzene and filtered hot through a silica pad. Solvent was evaporated and the solid material was macerated in the mixture of 100 ml dioxane and 50 ml acetonitrile under reflux. Final purification was done by sublimation. White powder. Yield: 11,8 g (72%). (ESI-MS: 693).

**Compound I-5** - **6,6'-([1,1':2',1"-terphenyl]-2,4"-diyl)bis(2,4-diphenyl-1,3,5-triazine)**

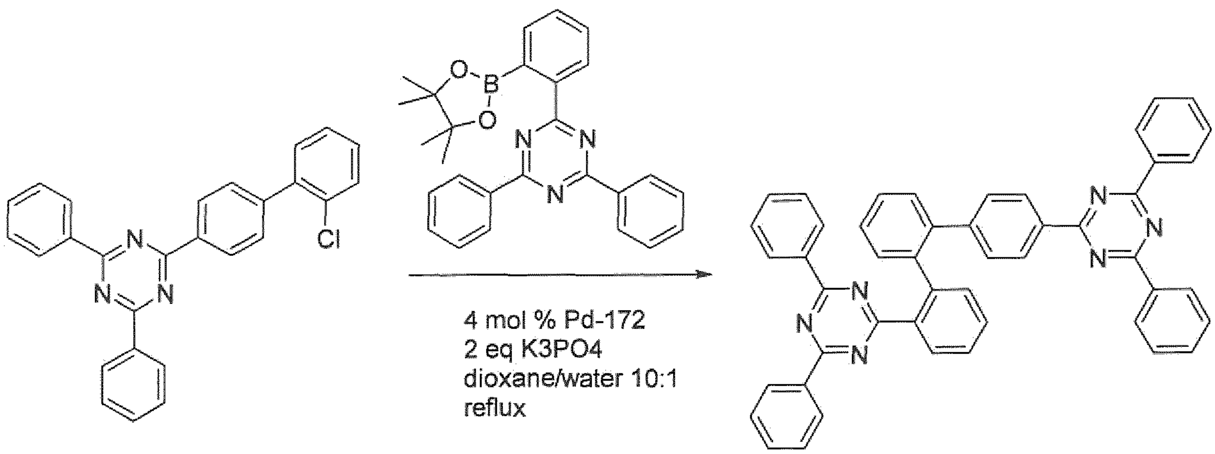


3 Neck round-bottom flask was flushed with nitrogen and charged with 2-(2'-chloro-[1,1'-biphenyl]-4-yl)-4,6-diphenyl-1,3,5-triazine (CAS 2243925-15-5) 1 eq (10,0 g), 2,4-diphenyl-6-(2-(4,4,5,5-tetramethyl-1,3,2-dioxaborolan-2-yl)phenyl)-1,3,5-triazine (CAS 1696425-27-0) 1,1 eq (11,4 g), chloro(crotyl)(2-dicyclohexylphosphino-2',6'-dimethoxybiphenyl)palladium(II) (CAS 1798781-99-3 ) 0,04 eq (0,6 g), potassium phosphate (CAS 7778-53-2) 2 eq (10,1 g). Deaerated mixture of 400 ml dioxane and 40 ml water was added. Reaction was running at reflux under a nitrogen atmosphere for 3 days. After cooling down to room temperature thick gray suspension was formed. Raw solid material was filtered, washed with water. Then the product was dissolved in 600 ml chloroform and filtered through a silica pad. Solvent was evaporated and the solid material was macerated in acetone under reflux. Final purification was done by sublimation. White powder. Yield: 3,8 g (24%). (ESI-MS: 693).

**Compound I-1 - 3,3"-bis(4,6-diphenyl-1,3,5-triazin-2-yl)-1,1':3',1"-terphenyl. CAS# 2055848-18-3**

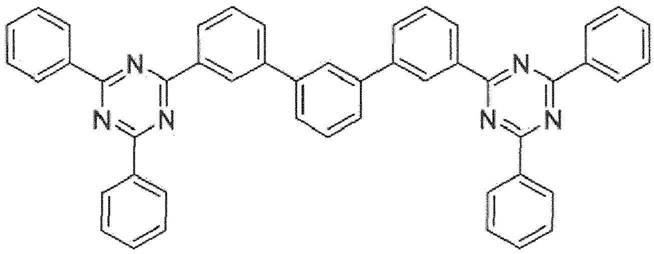

Synthesis described elsewhere (WO16204375).

Table 1: Properties of the synthesized compounds (DFΓ data Turbomole, SDC standard procedure (B3LYP-Gaussian / 6-31G*, gas phase))

| | HOMO [eV] | LUMO [eV] | E gap [eV] | Dipole [Debye] |
|---|---|---|---|---|
| ET-2 | -5,80 | -1,97 | 3,83 | 0,22 |
| I-1 | -6,07 | -1,82 | 4,26 | 0,23 |
| I-2 | -6,03 | -1,82 | 4,21 | 0,6 |
| I-3 | -6,05 | -1,85 | 4,20 | N/A |
| I-4 | -6,03 | -1,92 | 4,11 | 0,23 |
| I-5 | -6,00 | -1,85 | 4,14 | 0,65 |
| I-6 | -6,02 | -1,85 | 4,17 | 0,13 |

Table 2: Thermal and CV data of inventive compounds

| | Mp [°C] | Tg [°C] | Rate onset [°C] (test chamber; bottom sensor) |
|---|---|---|---|
| ET-2 | 358 | Not observed | 320 |
| I-1 | 294 | 110 | 284 |
| I-2 | Not observed | 118 | 264 |
| I-3 | 295 | 121 | 234 |
| I-4 | 348 | 138 | 297 |
| I-5 | 286 | 124 | 242 |
| I-6 | 278 | 128 | 242 |

Device experiments

**General procedure for fabrication of Bottom-emission OLEDs**

For bottom emission OLED device, an ITO/glass substrate was cut to a size of 150 mm × 150 mm × 0.7 mm, rinse with isopropyl alcohol for ~5 minutes and then with ultrasonically cleaned pure water for~ 5 minutes, and baked out in oven at 200°C for ~2h. Before organic material deposition it gets plasma treatment in vacuum chamber (typically N2 plasma).

The device was made by depositing a hole injection layer of HT-1 doped with D-1 onto an ITO anode placed on a glass substrate, followed by a undoped hole transport layer of HT-1. Subsequently, an electron blocking layer of HT-2 was deposited onto the HTL. Subsequently, a blue fluorescent emitting layer of emitter host Host-1 doped with Emitter-1 was deposited. On the emission layer, a layer made of ET-1 was deposited as a hole blocking layer. An electron transport layer made of the comparative compound ET-2 or the inventive compounds was codeposited with LiQ onto the HBL. Subsequently, Al cathode was deposited. All depositions were made by vacuum thermal evaporation.

OLED device structure - Stack: ITO, 90nm / HT-1:D-1 (3wt%), 10nm / HT-1, 128nm / HT-2, 5nm / Host-1:Emitter-1

(1vol%), 20nm / ET-1, 5nm / inventive compound I-1 to 1-6 or comparative compound ET-2:LiQ (50vol%), 3mm / Al, 100nm

Structures of tested compounds

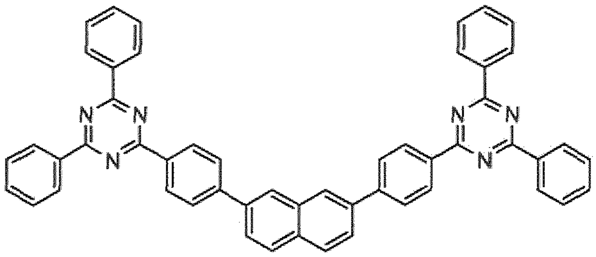

(ET-2) comparative example

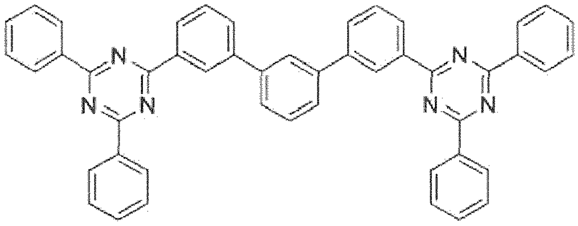

(I-1);

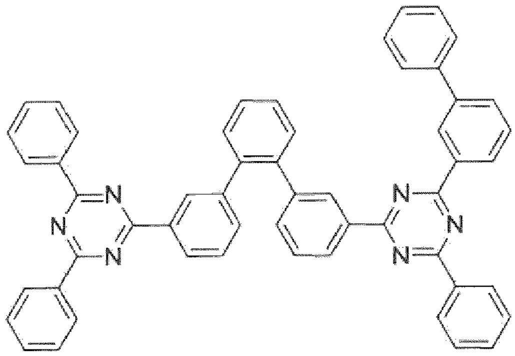

(I-2);

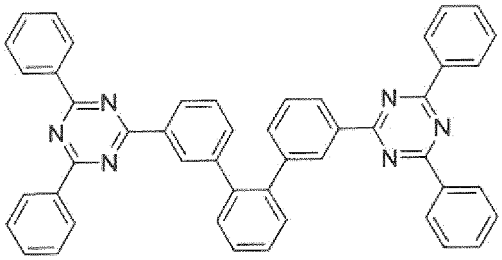

(I-3);

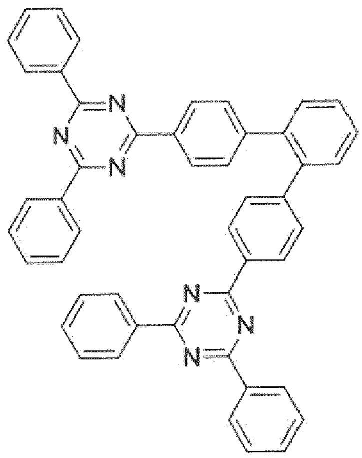

(I-4);

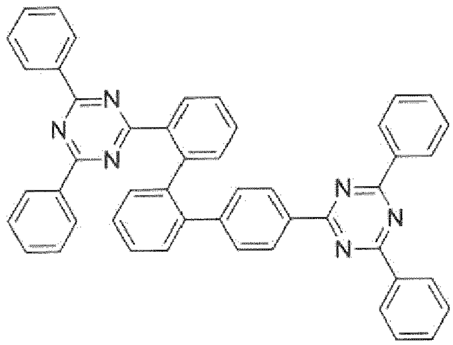

(I-5);

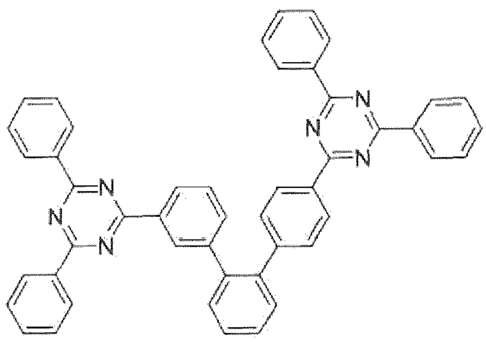

(I-6);

Table 3: List of compounds used

| | IUPAC name | Reference |
|---|---|---|
| HT-1 | N-([1,1'-biphenyl]-4-yl)-9,9-dimethyl-N-(4-(9-phenyl-9H-carbazol-3-yl)phenyl)-9H-fluoren-2-amine<br>CAS 1242056-42-3 | US2016322581 |
| D-x | 4,4',4"-((1E,1'E,1E")-cyclopropane-1,2,3-triylidenetris(cyanomethanylylidene)) tris(2,3,5,6-tetrafluorobenzonitrile)<br>CAS 1224447-88-4 | US2008265216 |
| HT-2 | N,N-di([1,1'-biphenyl]-4-yl)-3'-(9H-carbazol-9-yl)-[1,1'-biphenyl]-4-amine<br>CAS 1464822-27-2 | |
| Host-1 | CAS 2457172-82-4 | - |
| Emitter-1 | CAS 2482607-57-6 | - |

(continued)

| | IUPAC name | Reference |
|---|---|---|
| ET-1 | 4-([1,1'-biphenyl]-4-yl)-6-(3'-(9,9-dimethyl-9H-fluoren-4-yl)-[1,1'-biphenyl]-4-yl)-2-phenylpyrimidine<br>CAS 1955546-40-3 | WO2016105141 |
| LiQ | 8-Hydroxyquinolinolato-lithium<br>CAS 850918-68-2 | - |
| ET-2 | 2,7-bis(4-(4,6-diphenyl-1,3,5-triazin-2-yl)phenyl)naphthalene | - |
| | CAS 1453809-37-4 | |

Table 4: Performance of OLED Devices

| ETL | CIEy | Voltage, 15 mA/cm² [V] | CEff/CIEy, 15 mA/cm² [cd/A] |
|---|---|---|---|
| ET-2:LiQ (comparative) | 0.085 | 3.59 | 95.3 |
| I-1:LiQ | 0.085 | 3.72 | 98.0 |
| I-2:LiQ | 0.085 | 3.77 | 97.7 |
| I-4:LiQ | 0.084 | 3.58 | 97.1 |
| I-5:LiQ | 0.085 | 3.83 | 97.6 |
| I-6:LiQ | 0.084 | 3.72 | 97.3 |

As it can be seen from Table 4, organic light emitting devices in accordance with the present invention comprising a compound of Formula (1) in an electron transport layer thereof together with an alkali metal complex have improved efficiency.

The features disclosed in the foregoing description and in the dependent claims may, both separately and in any combination thereof, be material for realizing the aspects of the disclosure made in the independent claims, in diverse forms thereof.

## Claims

**1.** Organic light emitting device comprising an anode, a cathode, an electron transport layer, an emission layer, and an auxiliary electron transport layer, wherein

- the electron transport layer and the auxiliary electron transport layer are arranged between emission layer and the cathode;
- the auxiliary electron transport layer is arranged between the electron transport layer and the emission layer;
- the electron transport layer comprises a compound of formula (1)

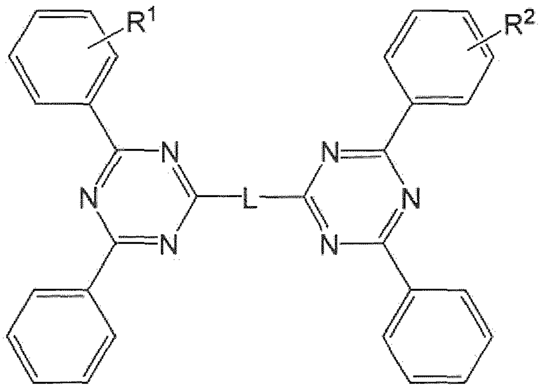
(I)

- $R^1$ and $R^2$ are independently selected from the group consisting of H, substituted or unsubstituted $C_6$ to $C_{18}$ aryl; substituted or unsubstituted $C_3$ to $C_{17}$ heteroaryl, CN, and CN-substituted $C_6$ to $C_{18}$ aryl;

  - wherein if one or more of $R^1$ and $R^2$ is substituted $C_6$ to $C_{18}$ aryl or $C_3$ to $C_{17}$ heteroaryl, the one or more substituent(s) are independently selected from the group consisting of D and $C_1$ to $C_4$ alkyl;

- L is selected from formulas (a) to (q)

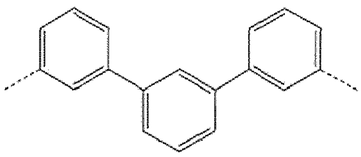
(a)
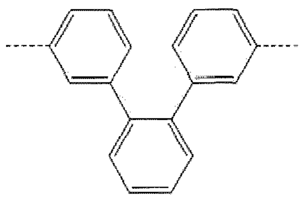
(b)
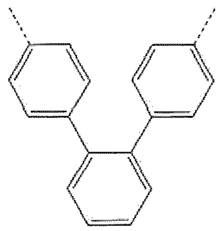
(c)
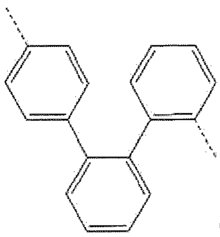
(d)
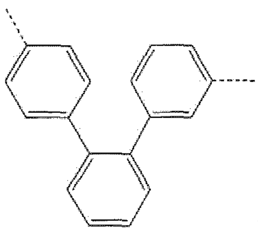
(e)
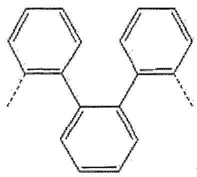
(f)
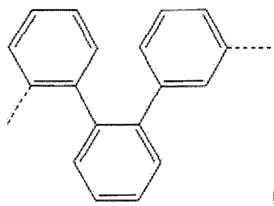
(g)

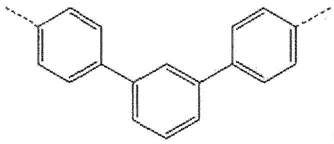 (h) 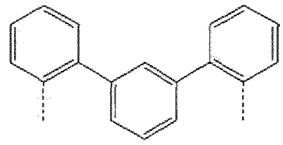 (i) 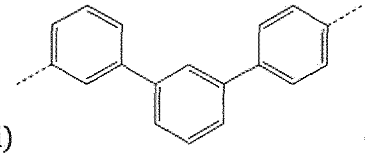 (j)

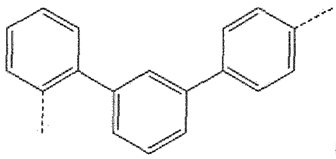 (k) 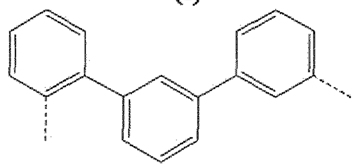 (l)

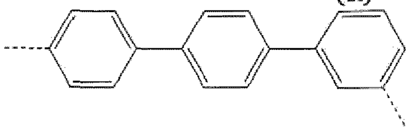 (m) 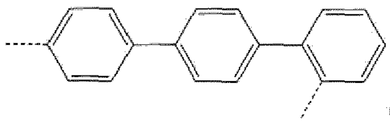 (n)

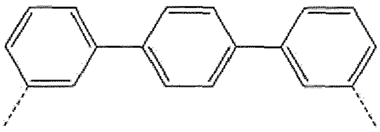 (o) 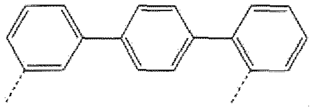 (p) 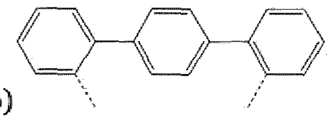 (q);

and

- the electron transport layer further comprises an alkali metal complex.

**2.** Organic light emitting device according to claim 1, wherein $R^1$ and $R^2$ are independently selected from the group consisting of H, phenyl, naphthyl, pyridyl, quinolinyl, CN, and CN-substituted phenyl.

**3.** Organic light emitting device according to claim 1 or 2, wherein $R^1$ is H and $R^2$ is selected from the group consisting of H, phenyl, naphthyl, pyridyl, quinolinyl, CN, and CN-substituted phenyl.

**4.** Organic light emitting device according to any of the preceding claims, wherein L is selected from (a) to (e)

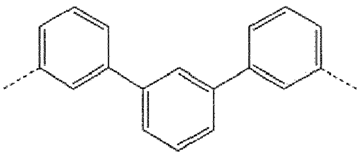 (a) 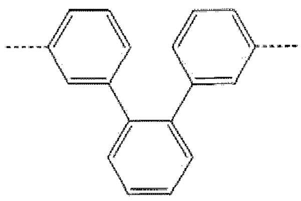 (b) 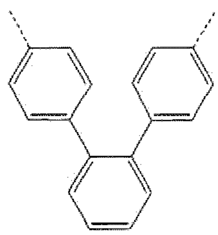 (c)

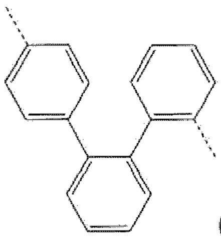 (d) 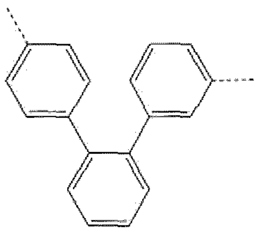 (e).

**5.** Organic light emitting device according to any of the preceding claims, wherein the compound of Formula I is selected from I-1 to I-25

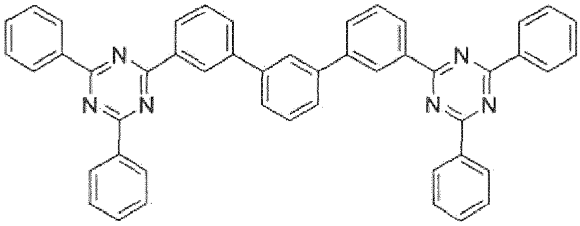

(I-1);

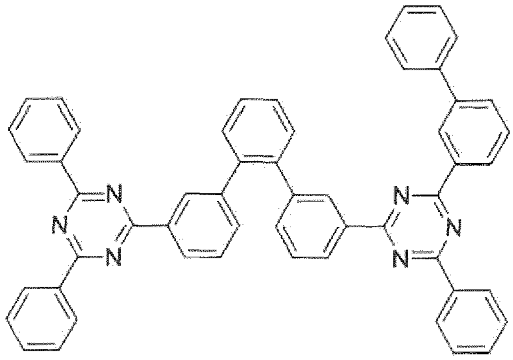

(I-2);

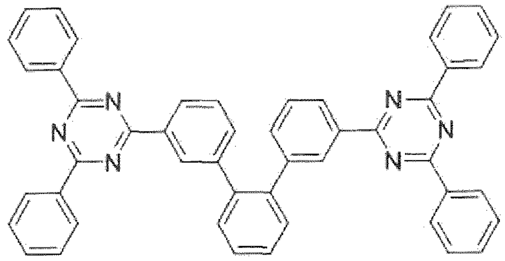

(I-3);

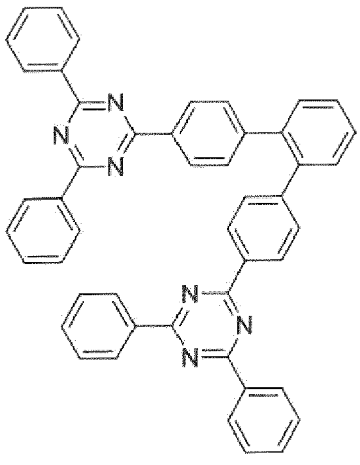

(I-4);

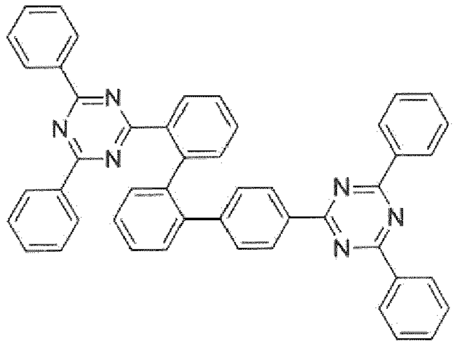

(I-5);

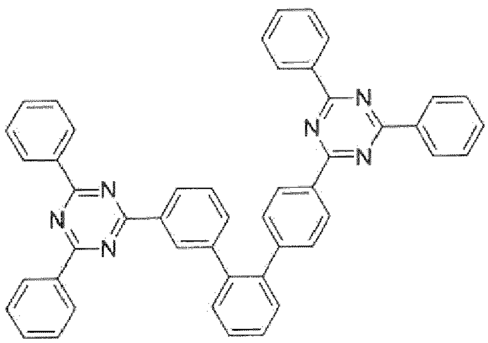

(I-6);

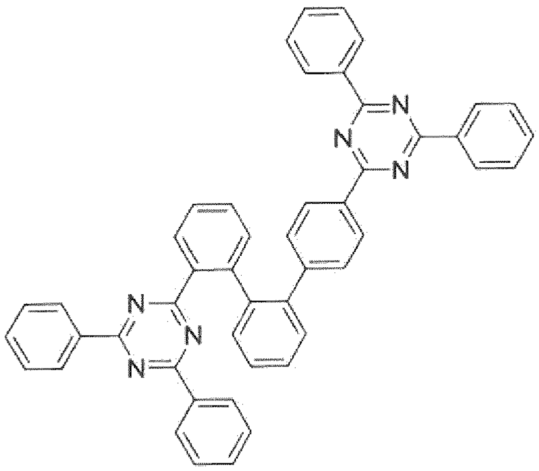

(I-7)

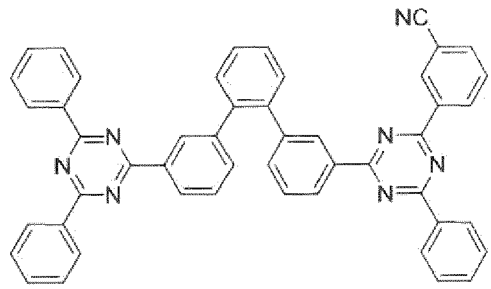

(I-8)

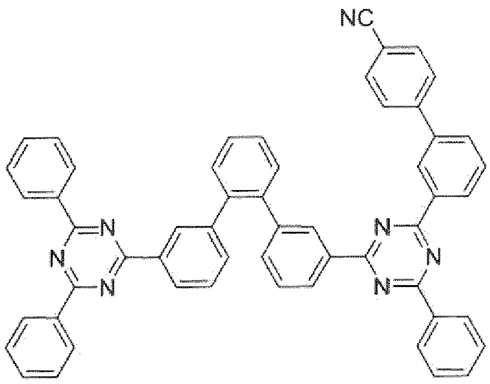

(I-9)

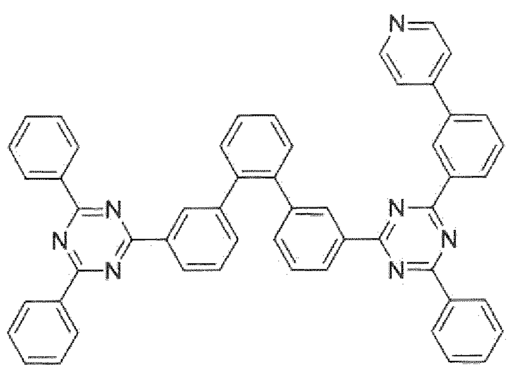

(I-10)

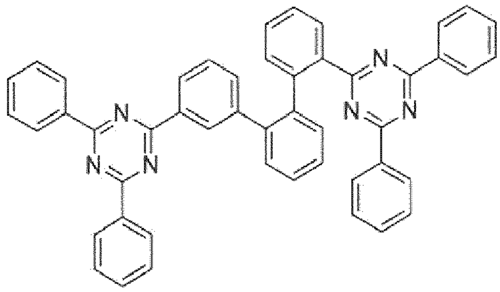
(I-11)

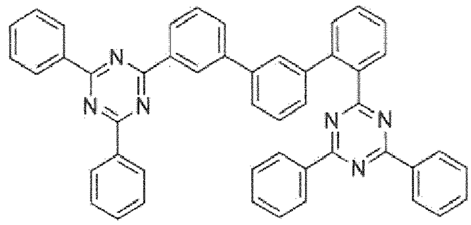
(I-12)

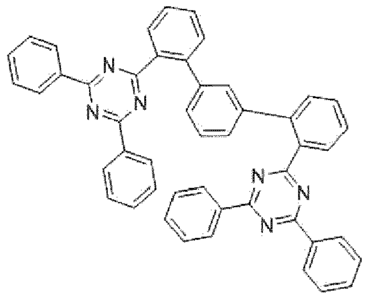
(I-13)

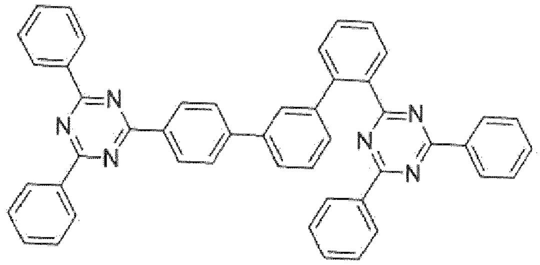
(I-14)

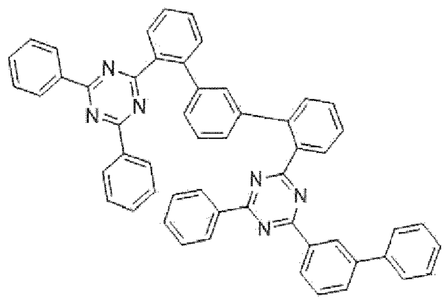
(I-15)

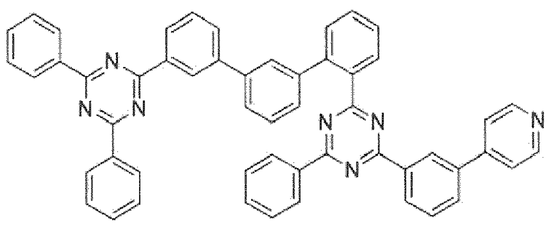
(I-16)

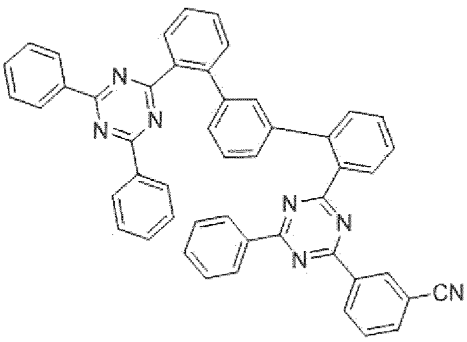
(I-17)

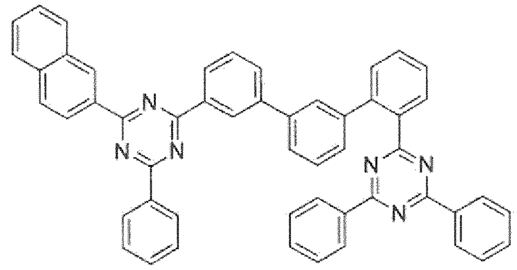
(I-18)

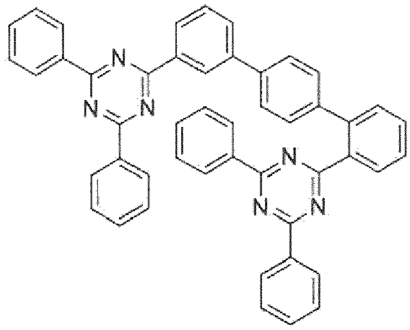

(I-19)

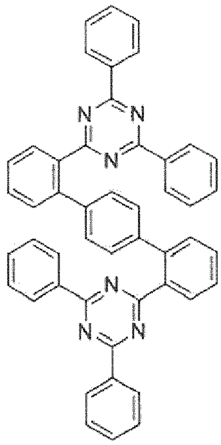

(I-20)

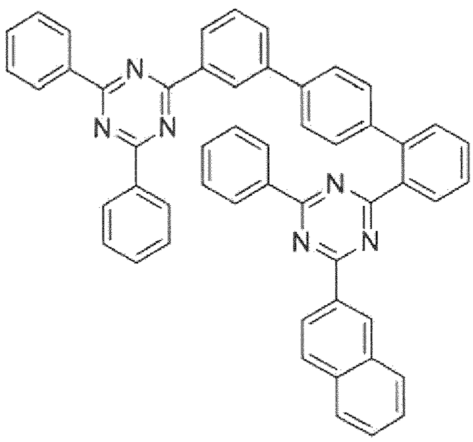

(I-21)

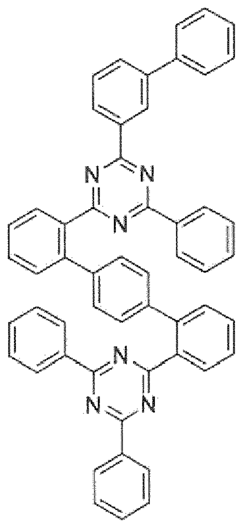

(I-22)

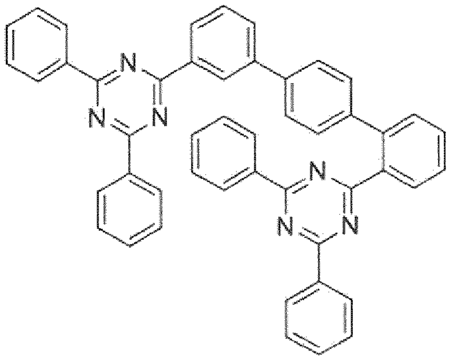

(I-23)

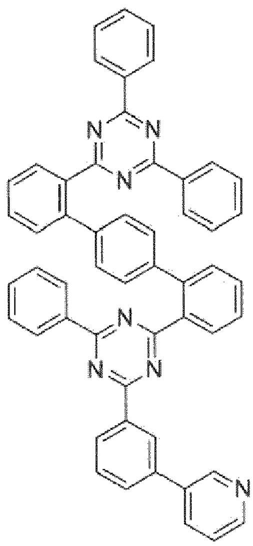

(I-24)

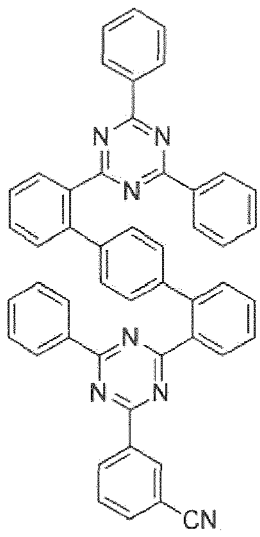

(I-25).

6. Organic light emitting device according to any of the preceding claims, wherein the alkali metal complex is a lithium-complex.

7. Organic light emitting device according to any of the preceding claims, wherein the alkali metal complex is 8-hydroxyquinolinolato-lithium.

8. Organic light emitting device according to any of the preceding claims, wherein the auxiliary electron transport layer comprises an auxiliary electron transport layer compound, wherein the auxiliary electron transport layer compound comprises 8 to 13 aromatic or heteroaromatic rings.

9. Organic light emitting device according to claim 8, wherein the auxiliary electron transport layer compound comprises at least one N-containing heteroaromatic ring.

10. Organic light emitting device according to any of the preceding claims, wherein

- the organic light emitting device further comprises an electron injection layer; and
- the electron injection layer is arranged between the electron transport layer and the cathode.

11. Display device comprising the organic light emitting diode according to any of the preceding claims.

12. Display device according to claim 11, wherein the display device is a television, a tablet, or a mobile phone.

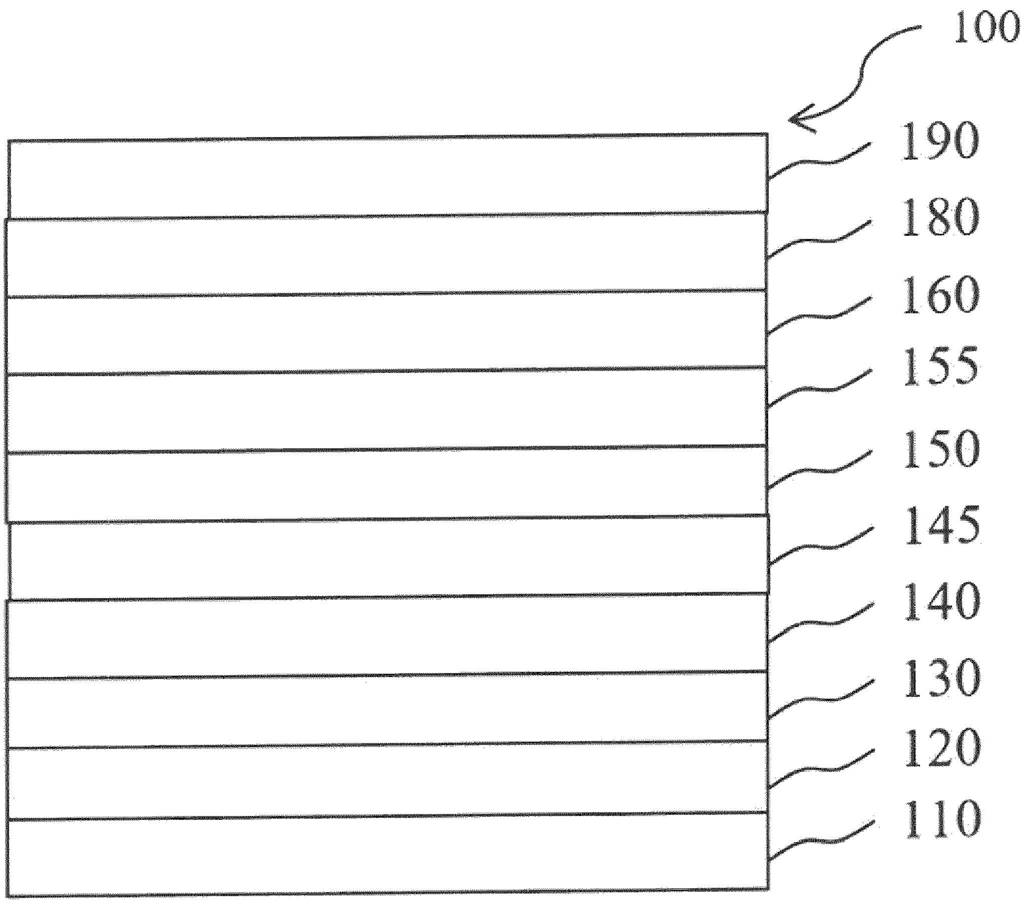


Fig. 1

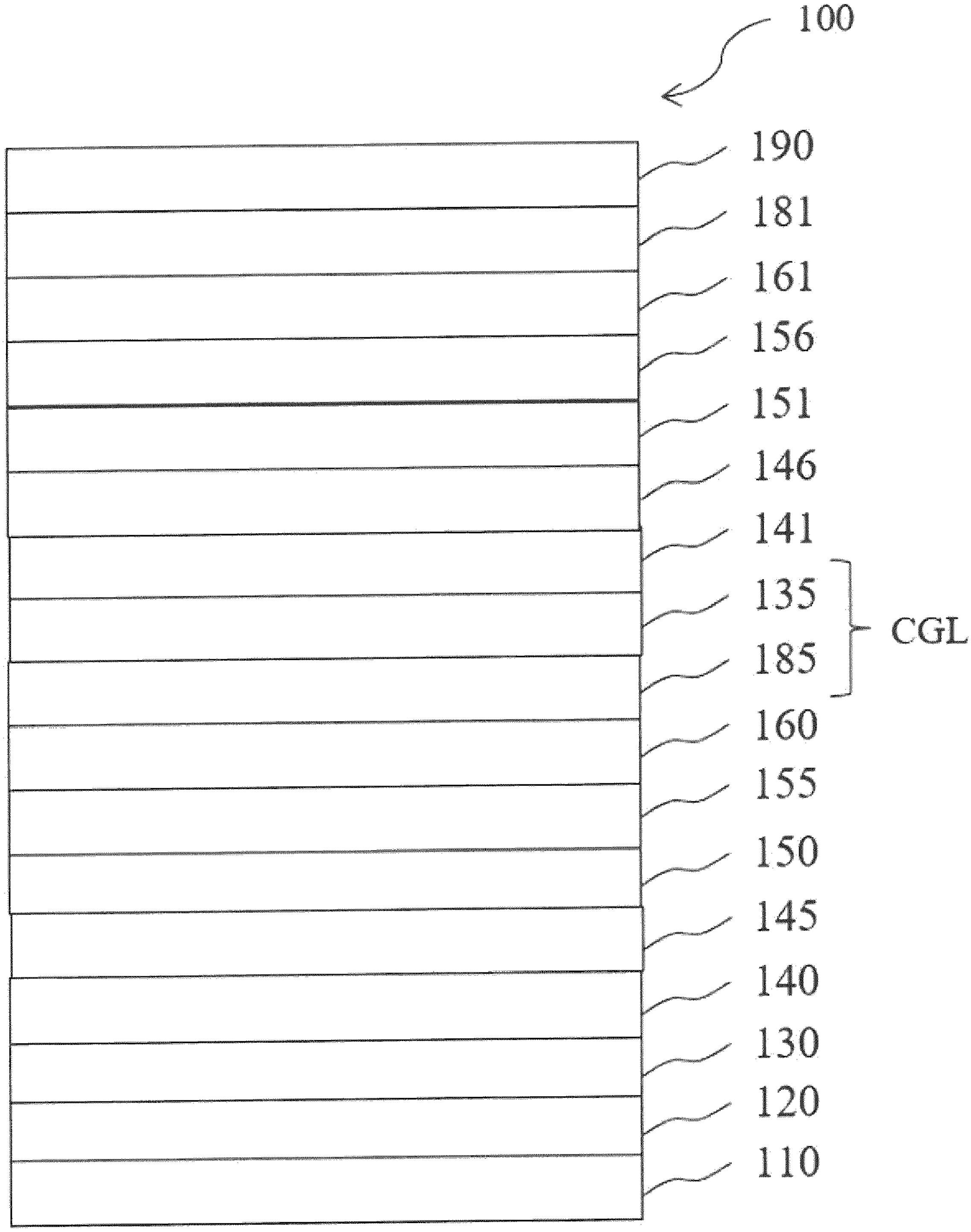
100
190
181
161
156
151
146
141
135
185
CGL
160
155
150
145
140
130
120
110

Fig. 2

Europäisches Patentamt
European Patent Office
Office européen des brevets

# EUROPEAN SEARCH REPORT

Application Number
EP 23 17 6122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2018 0044832 A (LG CHEMICAL LTD [KR]) 3 May 2018 (2018-05-03)<br>* page 49, paragraph 324 - page 50, paragraph 329; example 1 *<br>* page 45; compounds 1-1 *<br>----- | 1-12 | INV.<br>H10K85/60<br>C07D251/24<br><br>ADD.<br>H10K50/165<br>H10K50/16<br>H10K50/18 |
| A | WO 2023/048426 A1 (LG CHEMICAL LTD [KR]) 30 March 2023 (2023-03-30)<br>* the whole document *<br>----- | 1-12 | |

| TECHNICAL FIELDS SEARCHED (IPC) |
|---|
| H10K<br>C07D |

1

The present search report has been drawn up for all claims

| Place of search | Date of completion of the search | Examiner |
|---|---|---|
| The Hague | 10 November 2023 | Parashkov, Radoslav |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 17 6122

This annex lists the patent family members relating to the patent documents cited in the above-mentioned European search report. The members are as contained in the European Patent Office EDP file on
The European Patent Office is in no way liable for these particulars which are merely given for the purpose of information.

10-11-2023

| Patent document cited in search report | Publication date | Patent family member(s) | Publication date |
|---|---|---|---|
| KR 20180044832 A | 03-05-2018 | NONE | |
| WO 2023048426 A1 | 30-03-2023 | KR 102486972 B1 | 09-01-2023 |
| | | WO 2023048426 A1 | 30-03-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2722908 A1
- EP 1970371 A1
- WO 2013079217 A1
- WO 16204375 A
- US 2016322581 A
- US 2008265216 A
- WO 2016105141 A


### Non-patent literature cited in the description


- **YASUHIKO SHIROTA** ; **HIROSHI KAGEYAMA**. *Chem. Rev.*, 2007, vol. 107, 953-1010
- *CHEMICAL ABSTRACTS*, 2649455-14-9
- *CHEMICAL ABSTRACTS*, 2032365-30-1
- *CHEMICAL ABSTRACTS*, 1798781-99-3
- *CHEMICAL ABSTRACTS*, 7778-53-2
- *CHEMICAL ABSTRACTS*, 269410-07-3
- *CHEMICAL ABSTRACTS*, 23449-08-3
- *CHEMICAL ABSTRACTS*, 14221-01-3
- *CHEMICAL ABSTRACTS*, 584-08-7
- *CHEMICAL ABSTRACTS*, 2243925-15-5
- *CHEMICAL ABSTRACTS*, 1269508-31-7
- *CHEMICAL ABSTRACTS*, 1696425-27-0
- *CHEMICAL ABSTRACTS*, 1242056-42-3
- *CHEMICAL ABSTRACTS*, 1224447-88-4
- *CHEMICAL ABSTRACTS*, 1464822-27-2
- *CHEMICAL ABSTRACTS*, 2457172-82-4
- *CHEMICAL ABSTRACTS*, 2482607-57-6
- *CHEMICAL ABSTRACTS*, 1955546-40-3
- *CHEMICAL ABSTRACTS*, 850918-68-2
- *CHEMICAL ABSTRACTS*, 1453809-37-4